(19) Europäisches Patentamt
European Patent Office
Office européen des brevets

(11) Publication number: **0 074 861**
**B1**

(12) # EUROPEAN PATENT SPECIFICATION

(45) Date of publication of patent specification: **27.08.86**

(21) Application number: **82304886.3**

(22) Date of filing: **16.09.82**

(51) Int. Cl.⁴: **C 07 D 295/14,**
C 07 D 213/65,
C 07 D 213/70,
C 07 D 307/42,
C 07 D 307/46,
C 07 D 333/16, A 61 K 31/44,
A 61 K 31/445,
A 61 K 31/535, A 61 K 31/54

(54) **Aminocyclopentane esters and their preparation and pharmaceutical formulation.**

(30) Priority: **16.09.81 GB 8127982**
**29.10.81 GB 8132675**
**29.04.82 GB 8212489**
**06.05.82 GB 8213069**

(43) Date of publication of application:
**23.03.83 Bulletin 83/12**

(45) Publication of the grant of the patent:
**27.08.86 Bulletin 86/35**

(84) Designated Contracting States:
**AT BE CH DE FR IT LI LU NL SE**

(58) References cited:
**EP-A-0 032 432**
**GB-A-2 028 805**
**GB-A-2 075 503**
**GB-A-2 079 281**

**The file contains technical information submitted after the application was filed and not included in this specification**

(73) Proprietor: **GLAXO GROUP LIMITED**
**Clarges House 6-12 Clarges Street**
**London W1Y 8DH (GB)**

(72) Inventor: **Collington, Eric W.**
**103 Warren Way Digswell**
**Welwyn Hertfordshire (GB)**
Inventor: **Hallett, Peter**
**46 Walnut Tree Close Bassingbourn**
**Royston Hertfordshire (GB)**
Inventor: **Wallis, Christopher J.**
**The White House Noons Folly Newmarket Road**
**Royston Hertfordshire (GB)**
Inventor: **Wadsworth, Alan**
**9 Maple Way Melbourn**
**Royston Hertfordshire (GB)**
Inventor: **Hayes, Norman F.**
**'Braemar' 201 Whitehill Road**
**Hitchin Hertfordshire (GB)**
Inventor: **Bradshaw, John**
**22 Whitely Close Dane End**
**Ware Hertfordshire (GB)**
Inventor: **Carter, Malcolm**
**29 The Dourne**
**Ware Hertfordshire (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(74) Representative: **Skailes, Humphrey John et al**
**Frank B. Dehn & Co. Imperial House**
**15-19 Kingsway**
**London WC2B 6UZ (GB)**

**Description**

The endoperoxides prostaglandins $G_2$ and $H_2$ and thromboxane $A_2$ are naturally occurring reactive metabolites of arachidonic acid in human platelets. They are not only potent aggregatory agents but are also constrictors of vascular and bronchial smooth muscle, and therefore substances which antagonise their effects are of considerable interest in human medicine.

Our British Patent Specifications 2028805A, 2075503A and 2079281A and European Specification 0032432AI describe groups of aminocyclopentanes for use in medicine.

We have now found a new group of compounds which have shown endoperoxide and thromboxane antagonist activity, and are therefore of interest in the treatment of asthma and cardiovascular diseases.

The invention thus provides compounds of the general formula (1a) and (1b)

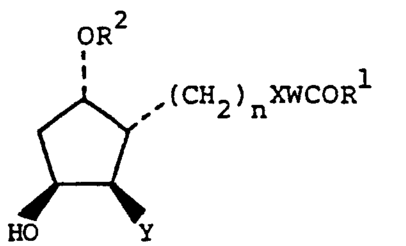
(1a)

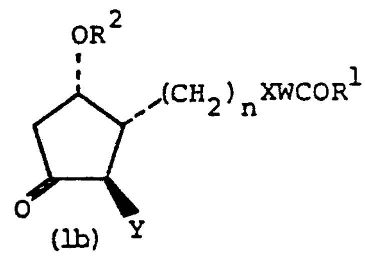
(1b)

wherein $R^1$ is

(a) $—AR^3$ where A is —O— or —S— and $R^3$ is phenyl [optionally substituted by $C_{1-4}$ alkyl (e.g. methyl, ethyl or t-butyl), $C_{1-4}$ alkoxy, (e.g. methoxy, ethoxy or butoxy, $C_{1-4}$ alkanoyl (e.g. acetyl), methylthio, methylsulphinyl, methylsulphonyl, halogen (e.g. chlorine or bromine, $—CO_2R^4$ [where $R^4$ is a hydrogen atom or $C_{1-4}$ alkyl (e.g. methyl or ethyl) or phenyl], $—NHCOR^4$ (e.g. acetamido, $—CONR^5R^6$ [where $R^5$ and $R^6$ may be the same or different and are each a hydrogen atom or $C_{1-4}$ alkyl (e.g. methyl or ethyl)], $C_{1-4}$ alkylsulphonylamino (e.g. $CH_3SO_2NH—$ or $C_2H_5SO_2NH—$), formyl, nitro, cyano, phenyl or $—NR^5R^6$ (e.g. amino, dimethylamino or diethylamino)];

(b) $—OCH_2COR^7$ where $R^7$ is phenyl [optionally substituted by a halogen atom (e.g. Cl or Br), $C_{1-4}$ alkyl (e.g. methyl, ethyl or t-butyl) or $C_{1-4}$ alkoxy, (e.g. methoxy, ethoxy or butoxy)] or $—NH_2$;

(c) $—A(CH_2)_mBR^5$ where m is 1—3 and B is —O— or —S—, provided that when m is 1, $R^5$ is not a hydrogen atom (e.g. $CH_3OCH_2O—$, $CH_3CH_2OCH_2O—$, $CH_3SCH_2O—$ or $HOC_2H_4O—$);

(d) $—A(CH_2)_pR^8$ where p is 2 or 3 and $R^8$ is an N-attached $C_{1-4}$ dialkylamino (e.g. dimethyl- or diethylamino), morpholino, piperidino, pyrrolidino, acetylamino or benzoylamino group;

(e) $—OCH(CH_2N(CH_3)_2)_2$;

$R^9$
|
(f) $—ACHCO_2R^5$ where $R^9$ is a hydrogen atom, methyl or phenyl; examples of suitable $R^5$ groups being methyl and ethyl;

(g) $—OCH_2OCOR^{10}$ where $R^{10}$ is $C_{1-4}$ alkyl (e.g. methyl or ethyl), methoxy or phenyl;

(h) $—OCH_2SCOR^{11}$ wherein $R^{11}$ is $C_{1-4}$ alkyl (e.g. methyl or ethyl);

(i) pyridinyloxy or pyridinylthio;

(j) 1-(acetyloxy)ethoxy, (acetyloxy)phenylmethoxy, tetrahydro-5-oxo-2-furanyloxy, tetrahydro-2-oxo-3-furanyloxy, triphenylmethoxy or diphenylmethoxy; or

(k) $—OR^{12}$ where $R^{12}$ is $C_{3-6}$ alkenyl (e.g. propenyl or butenyl), $C_{5-7}$ cyloalkyl (e.g. cyclopentyl, cyclohexyl or cycloheptyl) optionally substituted by one or more $C_{1-4}$ alkyl (e.g. methyl, ethyl or t-butyl) groups, $—CH_2CCl_3$ or furanylmethyl;

n is 1 or 2;

W is straight or branched $C_{1-7}$ alkylene;

X is cis or trans —CH=CH— or $—CH_2CH_2—$;

Y is a saturated heterocyclic amino group (attached to the cyclopentane ring via the nitrogen atom) which has 5—8 ring members and (a) optionally contains in the ring —O—, —S—, $—SO_2—$, or $NR^{13}$ (wherein $R^{13}$ is a hydrogen atom, $C_{1-7}$ alkyl or aralkyl having a $C_{1-4}$ alkyl portion); and/or (b) is optionally substituted by one or more $C_{1-4}$ alkyl groups;

$R^2$ is (i) straight or branched $C_{1-5}$ alkyl substituted by (a) phenyl [optionally substituted by $C_{1-6}$ alkyl, $C_{5-7}$ cycloalkyl, phenylalkyl having a $C_{1-3}$ alkyl portion, thienyl, phenyl (optionally substituted by $C_{1-4}$ alkyl, $C_{1-4}$ alkoxy or phenyl), benzoyl (optionally substituted by $C_{1-4}$ alkyl, $C_{1-4}$ alkoxy, halogen, trifluoromethyl or phenyl) or $C_{5-7}$ cycloalkanoyl], (b) thienyl [optionally substituted by $C_{5-7}$ cycloalkyl or phenyl (optionally substituted by $C_{1-3}$ alkyl, $C_{1-3}$ alkoxy or halogen)], or (c) naphthyl (optionally substituted by $C_{1-4}$ alkyl or

$C_{1-4}$ alkoxy, or (ii) cinnamyl (optionally substituted by benzoyl); and the physiologically acceptable salts and solvates thereof.

The structural formulae herein are to be understood to include the enantiomers of each of the compounds concerned as well as mixtures of the enantiomers including racemates, even though the precise structure as set out only relates to one enantiomer.

The amino group Y enables the compounds to form salts with acids. Compounds of formula (1a) can form salts with inorganic or organic acids, e.g. hydrochlorides or maleates, and compounds of formula (1b) can form salts with organic acids. Also, when the group $R^1$ contains a —COOH group, salts may be formed with bases. Examples of such salts are alkali metal (e.g. sodium), alkaline earth metal (e.g. calcium) and amine (e.g. piperazine) salts.

The heterocyclic amino group Y may for example have a 5,6 or 7-membered ring, e.g. pyrrolidino, piperidino, morpholino, piperazino, thiomorpholino, 1,1-dioxothiomorpholino, homomorpholino and hexamethyleneimino. Examples of the optional substituents which may be present on a second nitrogen atom in the ring are methyl, ethyl and benzyl. The carbon atoms of the heterocyclic rings may for example be substituted by methyl or ethyl. Y is preferably a morpholino or piperidino group.

In the group $—(CH_2)_nXWCOR^1$, n is preferably 2. X is preferably a cis —CH=CH— group. The W group may for example contain 1—5 carbon atoms in a straight or branched chain and is preferably $—CH_2CH_2CH_2—$ when n is 1, and $—CH_2CH_2—$ or $—CH_2CH_2CH_2CH_2—$ when n is 2.

In the $R^1$ groups, A (where present) is preferably —O—. In $R^1$ groups of the type (a), $R^3$ is preferably phenyl (optionally substituted by $C_{1-4}$ alkyl, $C_{1-4}$ alkoxy, $C_{1-4}$ alkanoyl, methylsulphonyl, $—COOR^4$ (where $R^4$ is a hydrogen atom or $C_{1-4}$ alkyl), $—NHCOR^4$ (where $R^4$ is $C_{1-4}$ alkyl) or $—CONR^5R^6$). In $R^1$ groups, of the type (b), $R^7$ is preferably $—NH_2$, phenyl or halophenyl. In $R^1$ groups of the type (c), m is preferably 1 or 2 and $R^5$ is preferably $C_{1-4}$ alkyl. In $R^1$ groups of the type (d), $R^8$ is preferably $C_{1-4}$ dialkylamino, morpholino or acetylamino. In $R^1$ groups of the type (f), $R^9$ is preferably methyl and $R^5$ is preferably $C_{1-4}$ alkyl.

Particularly preferred $R^1$ groups are $—OCH_2OCOCH_3$, $—OCH_2SCH_3$, $—OCH_2CH_2CH_2NHCOCH_3$, $—OCH_2CONH_2$, 4-acetamidophenoxy and allyloxy.

When $R^2$ is a substituted alkyl group, the alkylene portion may for example contain 1—3 carbon atoms (e.g. methylene, ethylene or propylene) and is preferably a methylene group.

In $R^2$ groups of the type (i)(a), the phenyl group may be substituted by, for example, methyl, ethyl, t-butyl, cyclohexyl, benzyl, phenethyl, phenyl (optionally substituted by methyl, ethyl, methoxy or butoxy), benzoyl (optionally substituted by methyl, ethyl, methoxy, butoxy, chlorine or bromine) or cyclohexanoyl groups.

In $R^2$ groups of the type (i)(b), the thienyl group may be substituted by, for example, methyl, ethyl, methoxy, ethoxy, cyclohexyl or phenyl (optionally substituted by methyl, ethyl, methoxy, ethoxy, chlorine or bromine) groups.

$R^2$ is preferably a benzyl group in which the phenyl group is substituted by thienyl or phenyl (which phenyl group may itself be optionally substituted by $C_{1-4}$ alkyl or $C_{1-4}$ alkoxy).

Particularly preferred $R^2$ groups are benzyl groups in which the phenyl portion is substituted (preferably in the para-position) by a phenyl, tolyl or methoxyphenyl substituent.

A particularly preferred group of compounds has the formula (1a) or (1b) in which:

$R^1$ is $—OCH_2OCOCH_3$, $—OCH_2SCH_3$, $OCH_2CH_2CH_2NHCOCH_3$, $—OCH_2CONH_2$, 4-acetamidophenoxy, or allyloxy,

W is $—CH_2CH_2—$

n is 2

X is cis —CH=CH—,

Y is morpholino or piperidino in compounds of formula (1b) or is piperidino in compounds of formula (1a), and

$R^2$ is benzyl in which the phenyl group is substituted by phenyl, tolyl or methoxyphenyl

and the physiologically acceptable salts and solvates thereof.

In general, the compounds of formula (1) in which the carbon atom carrying the $—(CH_2)_nXWCOR^1$ group is in the R— configuration (and mixtures containing this isomer) are preferred. Compounds of formula (1a) are also generally preferred.

Compounds of formula (1) inhibit blood platelet aggregation and bronchoconstriction. A test to determine inhibition of blood platelet aggregation is as described by G.V. Born in Nature *194,* 927—929 (1962) except in that collagen is used instead of ADP as the pro-aggregatory agent. Alternatively, starved guinea-pigs are dosed orally with the compound to be tested in a suitable vehicle. Platelet rich plasma is prepared from each animal and aggregation to a range of collagen concentrations is measured after the method of Born (Nature *194,* 927—929, (1962)). Collagen concentration-effect curves for each sample of plasma are calculated and results are expressed as the shift of the curves following treatment with the compound.

The ability of the compounds of the invention to inhibit bronchoconstriction is determined either in the anaesthetized guinea pig by measuring the effect of the compound to be tested on the dose response curve of the bronchoconstrictor [1R-[1α, 4α, 5β(Z), 6α-(1E, 3S*)]]-7-[6-(3-hydroxy-1-octenyl)-2-oxabicyclo[2,2,1]hept-5-yl]-5-heptenoic acid (U—46619), or by the test described by K.M. Lulich *et al* in British Journal of Pharmacology *58,* 71—79 (1976) except guinea pig lung is used instead of cat lung.

The compounds are thus of interest in the treatment of asthma, and as inhibitors of platelet aggregation and thrombosis for use in renal dialysis and the treatment and prevention of occlusive vascular diseases such as arteriosclerosis, atherosclerosis, peripheral vascular disease, cerebral vascular disease including transient ischaemic attacks, stroke, pulmonary embolism, diabetic retinopathy, post operative thrombosis, angina and myocardial infarction. They may be formulated in conventional manner for use, with one or more pharmaceutical carriers.

For oral administration, the pharmaceutical composition may take the form of, for example, tablets, capsules, powders, solutions, syrups, or suspensions prepared by conventional means with acceptable excipients.

The compounds may be formulated for parenteral administration by bolus injections or continuous infusion. Formulations for injections may be presented in unit dosage form in ampoules, or in multi-dose containers, with an added preservative. The compositions may take such forms as suspensions, solutions or emulsions in oily or aqueous vehicles, and may contain formulatory agents such as suspending, stabilising and/or dispersing agents. Alternatively, the active ingredient may be in powder form for reconstitution before use with a suitable vehicle, e.g. sterile pyrogen-free water.

For administration by inhalation the compounds are conveniently delivered in the form of an aerosol spray presentation from pressurised packs or a nebuliser, or as a cartridge from which the powdered composition may be inhaled with the aid of a suitable device. In the case of a pressurised aerosol the dosage unit may be determined by providing a valve to deliver a metered amount.

For use as antithrombotic agents, the compounds are preferably administered orally, for example in amounts of 0.05 to 10 mg/kg body weight, 1 to 4 times daily.

For use in the treatment of asthma, the compounds may also be administered orally in amounts of 0.05 to 10 mg/kg body weight, 1 to 4 times daily; preferably however they are administered by inhalation at doses varying from 0.3 to 30 mg, 1 to 4 times daily. The compounds may be used in combination with other anti-asthmatic agents.

The precise dose administered will of course depend on the age and condition of the patient.

Suitable methods for preparing the compounds of the invention are described below, the groups $R^1$, $R^2$, $R^3$, $R^5$, A, B, W, X and Y being as defined above except where otherwise indicated.

(a) The compounds of the invention may be prepared by esterification of the corresponding carboxylic acid, i.e. a compound in which $R^1$ represents a hydroxyl group. Conventional esterification methods may be used.

For example, compounds in which $R^1$ is a group of the type a, c (other than those in which m is 1), d, e, f, and k may be prepared by treating a reactive derivative of the corresponding carboxylic acid with an appropriate alcohol or thiol $R^1H$. The reactions may for example be carried out at room temperature using a solvent such as ketone (e.g. methylethyl ketone or acetone) or acetonitrile and, where appropriate, in the presence of pyridine.

The reactive derivative is conveniently a mixed anhydride of the acid, formed for example by treatment of the acid with a chloroformate in the presence of a suitable base, e.g. triethylamine or pyridine at −10°C.

The chloroformate may for example by a $C_{1-6}$ alkyl (e.g. iso-butyl), aryl (e.g. phenyl) or aralkyl (e.g. benzyl) chloroformate.

The same group of esters may also be prepared by first reacting the corresponding carboxylic acid with dicyclohexylcarbodiimide in the presence of 4-pyrrolidinopyridine, and then treating the product with the alcohol or thiol $R^1H$. This reaction is conveniently performed at room temperature in a solvent such as ether or $CH_2Cl_2$.

Again for example, compounds in which $R^1$ is a group of the type b, c, d, f, g, h, or j and A (when present) is —O— may be prepared by reacting the corresponding carboxylic acid with an appropriate halide $R^{14}$ Hal, where Hal represents halogen and $R^{14}$ is as just defined for $R^1$, excluding the terminal —O—. The reaction is carried out in the presence of a suitable base, e.g. potassium t-butoxide or potassium carbonate or a sterically hindered amine such as N,N-diisopropylethylamine, triethylamine or dicyclohexylamine in a suitable solvent (such as acetonitrile, dimethylformamide, $CH_2Cl_2$ or a ketone, e.g. methylethyl ketone or acetone), for example at a temperature from 0°C to room temperature.

This latter reaction may also be used to prepare compounds in which $R^1$ is a group of type (c) in which A is —S— and m is 1, by reacting the corresponding thioacid (i.e. in which $R^1$ is —SH) with a halide $R^5BCH_2$—Hal. The thioacid starting material may be prepared *in situ* by treating a reactive derivative of the corresponding carboxylic acid (e.g. a mixed anhydride, as above) with a hydrosulphide (e.g. NaHS).

Compounds in which $R^1$ is a group of the type (f) where A is —O— may also be prepared by reacting the corresponding carboxylic acid with the appropriate diazoalkane in a solvent such as dioxan in the presence of a salt such as copper (II) chloride, for example at room temperature.

Compounds in which $R^1$ is a group of the type (i) where A is —S— may also be prepared by reacting the corresponding carboxylic acid with the appropriate pyridyldisulphide in the presence of triphenylphosphine in a solvent such as benzene, for example at room temperature.

Many of the carboxylic acids corresponding to the esters of formula (1b) required as starting materials for process (a), i.e. compounds of formula (2)

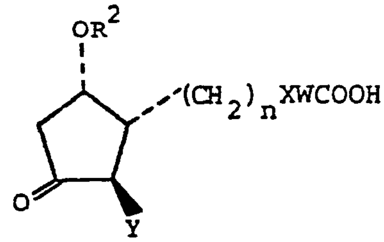

(2)

are described in British Patent Specifications 2028805A, 2070591A and 2075503A and those containing other $R^2$ groups may be prepared by the same general methods, using starting materials containing the desired $R^2$ group. Carboxylic acids required as starting materials for the preparation of compounds of formula (1a) by process (a) may be prepared by selective reduction of the corresponding acid of formula (2) using for example a selective reducing agent such as lithium tri-sec-butylborohydride in a solvent such as tetrahydrofuran at a temperature from 0° to −78°C. Where a carbonyl group is present in $R^2$ this will need to be in a protected state during this reaction. Conventional protection methods may be used, with regard to the reducing conditions.

(b) The compounds of the invention may also be prepared by reduction of a corresponding compound in which X is an acetylene group. Suitable methods of reduction include using hydrogen in the presence of a catalyst, e.g. palladium on a support (e.g. $CaCO_3$ or $BaSO_4$) and poisoned for example by lead or quinoline. Suitable solvents include ethyl acetate and methanol. This reaction is particularly suitable for preparing compounds in which X is cis —CH=CH—.

The acetylenes required as starting materials may be prepared by first brominating (e.g. with bromine in $CH_2Cl_2$) a compound of formula (3)

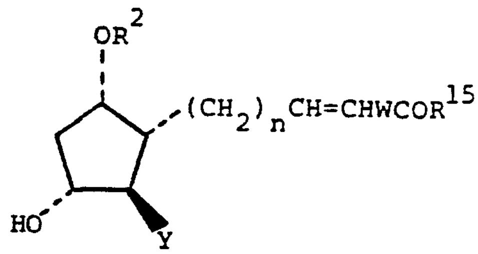

(3)

(where $R^{15}$ is $C_{1-6}$ alkoxy, e.g. methoxy) to give the corresponding compound in which X is —CHBr.CHBr—. The latter dibromo compound is then dehydrobrominated to form the acetylene group, for example in two stages, using potassium t-butoxide first at 0°C and then at room temperature. Hydrolysis of the resulting acetylene ester to the corresponding acid ($R^1$=OH) using a base such as NaOH at e.g. room temperature, followed by oxidation of the ring hydroxy group (using e.g. chromic acid in acetone at a low temperature e.g. −20°C to room temperature) gives a corresponding acid of formula (1b) in which X is an acetylene group and $R^1$ is —OH. Reduction of the product using e.g. lithium tri-sec-butylborohydride as described above for method (a) gives a corresponding compound of formula (1a) in which X is an acetylene group and $R^1$ is —OH. Esterification of the acetylene acids as described in method (a) then gives the required acetylene ester starting materials. The starting materials of formula (3) may be prepared by the methods generally described in British Patent Specifications 2028805A, 2070591A and 2075503A.

(c) Compounds in which $R^1$ is a group of type (a) in which $R^3$ is phenyl substituted by amino may be prepared by reduction of the corresponding compound in which $R^3$ is phenyl substituted by azido. The reduction may for example be effected with zinc and potassium dihydrogen phosphate in a suitable solvent, e.g. tetrahydrofuran.

(d) Compounds in which X is $—CH_2CH_2—$ may be prepared by catalytic hydrogenation of a corresponding compound in which X is —CH=CH—, using a catalyst such as palladium oxide. Alcohols such as ethanol are suitable solvents and the reaction may be performed at room temperature.

(e) Compounds of formula (1b) may be prepared by oxidising a corresponding hydroxy compound, e.g. a compound of formula (4)

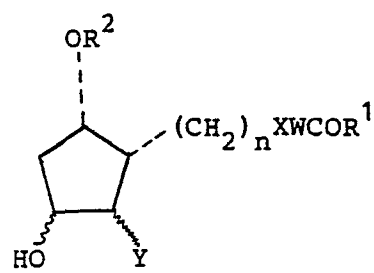

(4)

Suitable methods of oxidation include using a $Cr^{VI}$ oxidising reagent in a suitable solvent, e.g. chromic acid in acetone (e.g. Jones reagent, preferably used in the presence of a diatomaceous silica such as Celite) or $CrO_3$ in pyridine. These reagents are for example used at temperatures of −20°C to room temperature.

Other important methods include using an activated sulphur reagent, e.g. (i)N-chlorosuccinimide-dimethyl-sulphide complex in a suitable solvent (e.g. toluene or dichloromethane) at temperatures of for example −25 to 25°C, preferably at 0—5°C, (ii) a dialkylsulphoxide (e.g. dimethylsulphoxide) activated by a suitable electrophilic reagent (such as oxalyl chloride, acetyl bromide or thionyl chloride) in a suitable solvent (e.g. toluene or dichloromethane), e.g. at −70 to −20°C; dicyclohexylcarbodiimide can also be used as the electrophilic reagent (preferably in the presence of $CF_3COOH$ or its pyridinium salt) at for example −10°C using the same solvents, or (iii) pyridine-$SO_3$ complex in dimethylsulphoxide, preferably at 0°C to room temperature. When Y is in the α-configuration conditions should be chosen to effect epimerisation, either at the same time or after oxidation.

The esters of formula (4) may be prepared by esterification of the corresponding carboxylic acid in which $R^1$ is a hydroxyl group, for example using the methods described above in connection with process (a).

The carboxylic acid starting materials may be prepared by the methods generally described in British Patent Specifications 2028805A, 2070591A and 2075503A.

Compounds of the formula (1b) in which the ester group is sensitive to oxidation are preferably prepared by the esterification process (a).

(f) Compounds of formula (1b) in which $R^1$ is $—OCH_2SCH_3$ may be prepared by simultaneous oxidation and esterification of the corresponding hydroxy carboxylic acid, i.e. a compound of formula (4) in which $R^1$ is —OH.

This reaction may be performed by treating the hydroxy carboxylic acid with dimethylsulphoxide in the presence of an electrophilic reagent, for example as described above for process (e), preferably using dicyclohexylcarbodiimide in the presence of pyridinium trifluoroacetate. The reaction is preferably carried out at room temperature.

(g) Where salts of compounds of formula (1) are desired such salts may be formed by conventional methods, for example by treatment with an acid or when $R^1$ contains a —COOH group, with a base. Salts of acids may be prepared by adding the acid to a solution of the compound of formula (1) in an organic solvent such as ether. Salts of bases may be prepared by adding the base (e.g. an amine such as piperazine) in a solvent such as ether.

When a specific enantiomer of formula (1) is required, starting materials having the desired stereochemical configuration should be used in the above processes. Such starting materials may for example be prepared from an enantiomeric bromohydrin as generally described in British Patent Specification 2075503A.

The following test results were obtained with the compounds of the invention.

Inhibition of Blood Platelet Aggregation

Starved guinea pigs were dosed orally (1mg/kg) with the compound to be tested in 1% $NaHCO_3$ solution. Platelet rich plasma was prepared from each animal and aggregation to a range of collagen concentrations (0.2 ug/ml—20 ug/ml) was measured after the method of Born (Nature *194.*, 927—929 (1962). Collagen concentration-effect curves for each sample of plasma were calculated. Thus for example the compounds of Examples 1a, 2a, 2b, 2c, 2i, 2j, 2l, 2m, 3a, 3b, 3c, 4, 5, 6a, 7, 9a, 10, 11, 12, 13, 14, 15, 16c, 16g, 16h, 16i, 17, 19a, 20, 23a, 23c, 24a, 24b, 25, 28 and 30 produced shifts of the curves greater than 2.Ox.

Toxicity

The compounds of the invention are non-toxic at therapeutically useful doses. Thus for example, the compounds of the examples produced no adverse effects when administered orally to guinea pigs at a dose of 1mg/kg.

The following examples illustrate the invention.

'Jones reagent' is a solution of chromic acid and sulphuric acid in water. A 2.67M solution contains $CrO_3$ (26.7g) and concentrated $H_2SO_4$ (23ml) made up to 100ml with water.

Temperatures are in °C. The following abbreviations are used: 'Dried' refers to drying with $MgSO_4$; T.L.C. — thin layer chromatography using $SiO_2$; PE — petroleum ether (boiling at 40—60° unless otherwise stated); THF — tetrahydrofuran; DMF — dimethylformamide; ER — ether; EA — ethyl acetate; DMSO — dimethylsulphoxide; IPA — isopropyl alcohol; DIBAL — diisobutylaluminium hydride. Chromatography was carried out using silica gel unless otherwise stated. The following abbreviations define the eluent used for the chromatography and T.L.C.

| | |
|---|---|
| (A) | 19:1 EA—$CH_2Cl_2$ |
| (B) | 9:1 ER-methanol |
| (C) | ER |
| (D) | 92:8 EA-methanol |
| (E) | EA |
| (F) | 9:1 EA-methanol |

| | |
|---|---|
| (G) | 19:1 ER-methanol |
| (H) | 4:1 ER—PE (b.p. 60—80°) |
| (I) | 49:1 ER-methanol |
| (J) | 2:1 ER—PE (b.p. 60—80°) |
| (K) | 2:1 ER—PE |
| (L) | 1:1 ER—PE (b.p. 60—80°) |
| (M) | 3:1 ER—PE |
| (N) | 4:1 ER—PE |
| (O) | 4:1 ER-methanol |
| (P) | 39:1 ER-methanol |
| (Q) | 3:1 EA—PE |
| (R) | 94:5:1 ER-methanol-$Et_3N$ |
| (S) | 1:1 ER—PE |
| (T) | 7:3 ER-isopentane |
| (U) | 65:35 ER—PE (b.p. 60—80°) |
| (V) | 7:3 ER—PE (b.p. 60—80°) |
| (W) | 9:1 ER—PE (b.p. 60—80°) |
| (X) | 4:1 PE (b.p. 60—80°)-EA |
| (Y) | 75:24:1 ER—PE—$CH_2Cl_2$ |
| (Z) | 3:1 ER—$CH_2Cl_2$ |
| (AA) | 5:4 PE (b.p. 60—80°):ER |
| (BB) | 13:9 EA—PE (b.p. 60—80°) |
| (CC) | 3:1 ER—PE (b.p. 60—80°) |
| (DD) | 13:7 PE (b.p. 60—80°)-EA |
| (EE) | 7:3 ER—PE |
| (FF) | 4:1 EA-methanol |
| (GG) | 24:1 ER-methanol |
| (HH) | 21:4 ER-methanol |
| (II) | 4:1 EA—PE (b.p. 60—80°) |
| (JJ) | 3:1 ER-isopentane |
| (KK) | 13:7 ER—PE (b.p. 60—80°) |
| (LL) | 99:1 EA-$Et_3N$ |
| (MM) | 199:1 EA-$Et_3N$ |
| (NN) | 97:3 EA—$CH_3OH$ |
| (OO) | 75:24:1 $CH_2Cl_2$-ER-$Et_3N$ |
| (PP) | 80:20:1 $CH_2Cl_2$-ER-$Et_3N$ |
| (QQ) | 66:34:1 $CH_2Cl_2$-ER-$Et_3N$ |
| (RR) | 95:5:0.5 EA—$CH_3OH$-$Et_3N$ |
| (SS) | 4:1 PE—ER |
| (TT) | 2:1 EA—$CH_3OH$ |
| (UU) | 1:1 EA—PE (b.p. 60—80°) |
| (VV) | 50:50:1 $CH_2Cl_2$—ER-$Et_3N$ |
| (WW) | 90:10:1 $CH_3OH$-$Et_3N$ |

The preparation of Intermediates 1—20 is described in British Patent Specification 2075503A.

Intermediate 1

[1α(Z), 2β,5α]-(±)-7-[5-[[(1,1'-Biphenyl)-4-yl]methoxy]-2-(4-morpholinyl)-3-oxocyclopentyl]-4-heptenoic acid.

Intermediate 2

[1α(Z),2β,5α]-(±)-7-[5-[[4'-Methoxy(1,1'-biphenyl)-4-yl]methoxy]-3-oxo-2-(4-thiomorpholinyl)cyclopentyl]-4-heptenoic acid, S-dioxide.

Intermediate 3

[1α(Z),2β,5α]-(±)-7-[2-(4-Morpholinyl)-3-oxo-5-[4-(phenylmethyl)phenylmethoxy]cyclopentyl]-4-heptenoic acid, compound with piperazine (2:1).

Intermediate 4

[1α(Z),2β,5α(E)]-(±)-7-[2-(4-Morpholinyl)-3-oxo-5-[(3-phenyl-2-propenyl)oxy]cyclopentyl]-4-heptenoic acid.

Intermediate 5

[1α(Z),2β,5α]-(±)-7-[5-[[(1,1'-Biphenyl)-4-yl]methoxy]-3-oxo-2-(4-thiomorpholinyl)cyclopentyl]-4-heptenoic acid.

Intermediate 6

[1α(Z),2β,3α,5α]-(±)-7-[3-Hydroxy-5-[[4′-methoxy(1,1′-biphenyl)-4-yl]methoxy]-2-(4-morpholinyl)cyclopentyl]-4-heptenoic acid.

Intermediate 7

[1α(Z),2β,3α,5α]-(±)-7-[3-Hydroxy-5-[[4′-methyl(1,1′-biphenyl)-4-yl]methoxy]-2-(4-morpholinyl)cyclopentyl]-4-heptenoic acid.

Intermediate 8

[1α(Z),2β,3α,5α(E)]-(±)-7-[3-Hydroxy-2-(4-morpholinyl)-5-[(3-phenyl-2-propenyl)oxy]cyclopentyl]-4-heptenoic acid.

Intermediate 9

[1α(Z),2β,3α,5α]-(±)-7-[5[[(1,1′-Biphenyl)-4-yl]methoxy]-3-hydroxy-2-(1-piperidinyl)cyclopentyl]-4-heptenoic acid.

Intermediate 10

[1R-[1α(Z),2β,5α]]-(−)-7-[5-[[(1,1′-Biphenyl)-4-yl]methoxy]-2-(4-morpholinyl)-3-oxocyclopentyl]-4-heptenoic acid.

Intermediate 11

[1α(Z),2β,3α,5α]-(±)-7-[5-[3-[(1,1′-Biphenyl)-4-yl]propoxy]-3-hydroxy-2-(4-morpholinyl)cyclopentyl]-4-heptenoic acid.

Intermediate 12

[1α(Z),2β,3α,5α]-(±)-9-[5-[[(1,1′-Biphenyl)-4-yl]methoxy]-3-hydroxy-2-(4-morpholinyl)cyclopentyl]-6-nonenoic acid.

Intermediate 13

[1α(Z),2β,3α,5α]-(±)-7-[3-Hydroxy-5-[[3′-methoxy(1,1′-biphenyl)-4-yl]methoxy]-2-(4-morpholinyl)cyclopentyl]-4-heptenoic acid.

Intermediate 14

[1α(Z),2β,3α,5α]-(±)-7-[3-Hydroxy-2-(4-morpholinyl)-5-[(2-phenylthien-4-yl)methoxy]cyclopentyl]-4-heptenoic acid.

Intermediate 15

[1α(Z),2β,3α,5α]-(±)-7-[3-Hydroxy-5-[[4′-methyloxy(1,1′-biphenyl)-4-yl]methoxy-2-(4-thiomorpholinyl)cyclopentyl]-4-heptenoic acid, S-dioxide.

Intermediate 16

[1α(Z),2β,3α,5α]-(±)-7-[3-Hydroxy-2-(4-morpholinyl)-5-[4-(thien-2-yl)phenylmethoxy]cyclopentyl]-4-heptenoic acid.

Intermediate 17

[1α(Z),2β,3α,5α]-(±)-7-[3-Hydroxy-2-(4-morpholinyl)-5-(2-naphthalenylmethoxy)cyclopentyl]-4-heptenoic acid.

Intermediate 18

[1α(Z),2β,3α,5α]-(±)-7-[5-[[(1,1′-Biphenyl)-4-yl]methoxy]-3-hydroxy-2-(4-morpholinyl)cyclopentyl]-4-heptenoic acid.

Intermediate 19

[1α(Z),2β,3β,5α]-(±)-7-[5-[[(1,1′-Biphenyl)-4-yl]methoxy]-3-hydroxy-2-(4-morpholinyl)cyclopentyl]-4-heptenoic acid.

Intermediate 20

(1α,2β,5α)-(±)-7-[5-[[(1,1′-Biphenyl)-4-yl]methoxy]-2-(4-morpholinyl)-3-oxocyclopentyl]-4-heptynoic acid.

Intermediate 21(a)

[1α(Z),2α,3α,5α]-(±)-(Acetyloxy)methyl 7-[5-[[(1,1′-Biphenyl)-4-yl]methoxy]-3-hydroxy-2-(4-morpholinyl)cyclopentyl]-4-heptenoate,
m.p. 69—71° from Intermediate 18 by the procedure described for the preparation of Example 3a (Method 1). Purification by chromatography (A) .I.R. ($CHBr_3$) 3480, 1760 $cm^{-1}$.

The following compounds were prepared by a similar procedure:—

(b) *(1α,2β,5α) - (±) - (Acetyloxy)methyl 7 - [5 - [[(1,1' - Biphenyl) - 4 - yl]methoxy] - 2 - (4-morpholinyl) - 3 - oxocyclopentyl] - 4 - heptynoate,* from Intermediate 20. Purification by chromatography (C) gave the *title compound* as an oil. I.R. (Neat) 1760, 1740cm$^{-1}$.

(c) *[1α(Z),2β,3α,5α] - (±) - (Acetyloxy)methyl 7 - [3 - Hydroxy - 2 - (4-morpholinyl) - 5 - [4 - (thien - 2 - yl)phenylmethoxy]cyclopentyl] - 4 - heptenoate,* from Intermediate 16. Purification by chromatography (F) gave the *title compound* as an oil. I.R. (Neat) 3430, 1765cm$^{-1}$.

Intermediate 22

[1α(Z),2β,3α,5α-(±)-Methoxymethyl 7-[5-[3-[(1,1'-Biphenyl)-4-yl]propoxy]-3-hydroxy-2-(4-morpholinyl)cyclopentyl]-4-heptenoate

Chloromethylmethyl ether (0.008 ml) was added to a mixture of Intermediate 11 (0.05 g) and dicyclohexylamine (0.02 ml) in dry DMF (0.75 ml). After 10 min saturated $NH_4Cl$ solution (10 ml) was added and the suspension extracted with EA (3×20 ml). The combined extracts were dried, filtered and evaporated to afford the *title compound* as an oil (0.042 g). I.R. (Neat) 3400, 1740cm$^{-1}$.

Intermediate 23

[1α(Z),2β,3α,5α]-(±)-(Methylthio)methyl 9-[5-[[(1,1'-Biphenyl)-4-yl]methoxy]-3-hydroxy-2-(4-morpholinyl)cyclopentyl]-6-nonenoate

From Intermediate 12 (1.25 g), chloromethyl methyl sulphide (0.61 ml) and diisopropylethylamine (1.5 ml) according to the method described for the preparation of Intermediate 22. Purification by chromatography (D) gave the *title compound* as an oil (0.37 g). I.R. ($CHBr_3$) 3430, 1733cm$^{-1}$.

Intermediates 24—27 were prepared by a similar procedure to that of Example 2a (Method 1).

Intermediate 24

[1α(Z),2β,3α,5α]-(±)-4-(Acetylamino)phenyl 7-[3-Hydroxy-5-[[3'-methoxy (1,1'-biphenyl)-4-yl]methoxy]-2-(4-morpholinyl)cyclopentyl]-4-heptenoate

From Intermediate 13 and 4-(acetylamino)phenol, in pyridine. Purification by chromatography initially using (E) then (F) gave the *title compound* as a foam. I.R. ($CHBr_3$) 3580, 3430, 1755, 1690, 1510cm$^{-1}$.

Intermediate 25

[1α(Z),2β,3α,5α]-(±)-4-Methylphenyl 7-[3-Hydroxy-2-(4-morpholinyl)-5-[(2-phenylthien-4-yl)methoxy]cyclopentyl]-4-heptenoate

From Intermediate 14 (0.26 g) and *p*-cresol (0.286 g). Purification by chromatography (D) gave the *title compound* as an oil (0.127 g). I.R. (Neat 3450, 1755cm$^{-1}$.

Intermediate 26

[1α(Z),2β,3α,5α]-(±)-4-(Methylsulphonyl)phenyl 7-[3-Hydroxy-5-[[4'-methyl(1,1'-biphenyl)-4-yl]methoxy]-2-(4-thiomorpholinyl)cyclopentyl]-4-heptenoate, S-dioxide

From Intermediate 15 (0.284 g) and 4-(methylsulphonyl)-phenol (0.473 g) using $CH_3CN$ as solvent. Purification by chromatography (G) gave the *title compound* as a foam (0.312 g). I.R. $CHBr_3$) 3580, 3550, 1760cm$^{-1}$.

Intermediate 27

[1α(Z),2β,3α,5α]-(±)-L-(−)-[1-Ethoxycarbonyl)ethyl] 7-[5-[[(1,1'-Biphenyl)-4-yl]methoxy]-3-hydroxy-2-(1-piperidinyl)cyclopentyl]-4-heptenoate

Prepared from Intermediate 9 (0.6 g) and L-ethyl lactate (0.385 g) using $CH_3CN$ as solvent. Chromatography (B) gave a mixture (1:1) of the *title compound* and [1α(Z),2β,3α,5α] - (±) - 2 - methylpropyl 7 - [5 - [[(1,1' - biphenyl) - 4 - yl]methoxy - 3 - hydroxy - 2 - (1-piperidinyl)cyclopentyl] - 4 - heptenoate as an oil (0.22g). I.R. ($CHBr_3$) 3600, 1730cm$^{-1}$.

Intermediate 28

[1α(Z),2β,3α,5α]-(±)-(Acetylthio)methyl 7-[5-[[(1,1'-Biphenyl)-4-yl]methoxy]-3-hydroxy-2-(1-piperidinyl)cyclopentyl]-4-heptenoate

From Intermediate 9 according to the procedure described for Example 8. Purification by chormatography (F). I.R. (Neat) 3360, 1740, 1705cm$^{-1}$.

Intermediate 29

[1α(Z),2β,3α,5α]-(±)-[2-(4-Bromophenyl)-2-oxoethyl] 7-[3-Hydroxy-2-(4-morpholinyl)-5-(2-naphthalenylmethoxy)cyclopentyl]-4-heptenoate

Intermediate 17 and 4-bromophenacyl bromide in an analogous manner to the preparation of Example 6a. Purification by chromatography initially using (G) followed by (B). I.R. (Neat) 3450, 1740, 1700cm$^{-1}$.

The preparation of Intermediate 30 is described in British Patent Specification 2028805A.

Intermediate 30

[1α(Z),2β,5α]-(±)-7-[5-[[(1,1′-Biphenyl)-4-yl]methoxy]-2-(4-morpholinyl)-3-oxocyclopentyl]-5-heptenoic acid

Intermediate 31

[1α(Z),2β,3β,5α]-(±)-7-[5-[[(1,1′-Biphenyl)-4-yl]methoxy]-3-hydroxy-2-(4-morpholinyl)cyclopentyl]-5-heptenoic acid, compound with piperazine, (2:1)

A solution of Intermediate 30 (1g) in THF (10 ml) was added dropwise to a stirred solution of lithium tri-sec-butylborohydride (1M in THF; 12 ml) under nitrogen at 0—5°. After 2.25h, the mixture was poured into 2N.$H_2SO_4$ (30 ml) and pH 6.5 phosphate buffer (50 ml) and washed with ER. The aqueous layer was adjusted to pH 6.5 with 2N NaOH and extracted with EA (3 × 50 ml). The combined extracts were dried, evaporated and the residue purified by chromatography (FF) to give a foam (0.81 g). A portion of the foam (0.105 g) in ER (30 ml) was treated with piperazine (20.3 mg) in ER (1.8 ml) to give the *title compound* as a solid (0.11 g), m.p. 121—127°.

The preparation of Intermediates 32—34 is described in British Patent Specification 2028805A.

Intermediate 32

[1α(Z),2β,3α,5α]-(±)-Methyl 7-[5-Hydroxy-2-(4-morpholinyl)-3-[(tetrahydro-2H-pyran-2-yl)oxy]cyclopentyl]-5-heptenoate

Intermediate 33

[1α(Z),2β,3α,5α]-(±)-7-[5-[[(1-1′-Biphenyl)-4-yl]methoxy]-3-hydroxy-2-(4-morpholinyl)cyclopentyl]-5-heptenoic acid

Intermediate 35

4-(Bromomethyl)phenyl-4-methoxyphenyl methanone

Benzoyl peroxide (1g) was added to a solution of 4-methoxyphenyl-4-methylphenyl methanone (22.6g) and N-bromosuccinimide (17.8g) in $CCl_4$ (100 ml). The mixture was heated under reflux for 3.25h, then cooled to 0°. The precipitated succinimide was filtered off, the filtrate evaporated and the residue purified by crystallisation from cyclohexane to give the *title compound* (8.9 g), m.p. 76—78°.

Intermediate 36

[1α(Z),2β,3α,5α]-(±)-7-[5-[4-(4-Methoxybenzoyl)phenylmethoxy]-2-(4-morpholinyl)-3-[(tetrahydro-2H-pyran-2-yl)oxy]cyclopentyl]-5-heptenoic acid

NaH (74% dispersion in oil, 0.74g) was added in portions over 4.5h to a solution of Intermediates 32 (0.93g) and 35 (2.77g) in DMF (10 ml). After a further 1h, the mixture was diluted with water (100 ml). Saturated $NH_4Cl$ solution (100 ml) was added, the mixture extracted with $CH_2Cl_2$ (4 × 50 ml), the combined extracts were dried and evaporated and the residue purified by chromatography using gradient elution (I) up to (HH) to give the *title compound* as an oil (1.1g). I.R. ($CHBr_3$) 3500, 1730—1700 (br), 1666cm$^{-1}$.

Intermediate 37

[1α(Z),2β,3α,5α]-(±)-7-[3-Hydroxy-5-[4-(4-methoxybenzoyl)-phenylmethoxy]-2-(4-morpholinyl)cyclopentyl]-5-heptenoic acid

A solution of Intermediate 36 (0.89 g) in acetone (40 ml) and saturated ethereal hydrogen chloride (15 ml) was kept at ambient temperature for 4.5h. The solvents were removed *in vacuo* and the residue in pH 6.5 phosphate buffer (50 ml) was extracted with $CH_2Cl_2$ (3 × 40 ml). The combined extracts were dried and evaporated and the residue purified by chromatography (HH) to give the *title compound* (0.33 g). I.R. ($CHBr_3$) 1730—1700 (br.), 1665cm$^{-1}$.

Intermediate 38

[1α(Z),2β,3α,5α]-(±)-2-Oxo-2-phenylethyl 7-[5-[[(1,1′-Biphenyl)-4-yl]methoxy]-3-hydroxy-2-(4-morpholinyl)cyclopentyl]-5-heptenoate

A suspension of Intermediate 33 (1g) in water (10 ml) was treated with potassium t-butoxide (0.234g) and the resulting solution was added to a solution of phenacyl bromide (0.6g) in ethanol (10 ml). The mixture was heated under reflux for 4h, poured into water and extracted with ER. The combined extracts were dried and evaporated and the residue purified by chromatography (G) to give the *title compound* as an oil (0.9g). I.R. ($CHBr_3$) 3540 (br.), 1740, 1705cm$^{-1}$.

Intermediate 39

[1α(Z),2β,3α,5α]-(±)-4-Acetylaminophenyl 7-[5-[[(1,1′-Biphenyl)-4-yl]methoxy]-3-hydroxy-2-(4-morpholinyl)cyclopentyl]-5-heptenoate

Isobutylchloroformate (0.82 ml) was added to a stirred solution of Intermediate 33 (1g) and triethylamine (1.16 ml) in acetone (10 ml) at −10°. After 0.6h, N-(4-hydroxyphenyl)acetamide (1.26 g) in acetone (24 ml) containing pyridine (7 drops) was added and stirring continued for a further 0.6h. The solvent was removed *in vacuo* and saturated $NH_4Cl$ solution (80 ml) added. The mixture was extracted with

EA (2 × 60 ml), the dried extracts evaporated and the residue purified by chromatography (B) to give the *title compound* as an oil (1.13 g). I.R. ($CHBr_3$) 3425, 1748, 1685cm$^{-1}$.

The preparation of Intermediates 40—42 is described in British Patent Specification 2070591A.

Intermediate 40

[1α(Z),2β,3α,5α]-(±)-7-[3-Hydroxy-2-(4-morpholinyl)-5-[4-phenylmethyl)phenylmethoxy]cyclopentyl]-5-heptenoic acid, hydrochloride

Intermediate 41

[1α(Z),2β,3α,5α]-(±)-7-[3-Hydroxy-2-(4-morpholinyl)-5-[[(1,1′:4′,1″-terphenyl)-4-yl]methoxy]cyclopentyl-5-heptenoic acid, hydrochloride

Intermediate 42

[1α(Z),2β,3α,5α]-(±)-7-[3-Hydroxy-5-[[(4′-methoxy(1,1′-biphenyl)-4-yl]methoxy]-2-(4-morpholinyl)cyclopentyl]-5-heptenoic acid, hydrochloride

Intermediate 43

[1α(Z),2β,3α,5α]-(±)-[4-(Aminocarbonyl)phenyl] 7-[3-Hydroxy-5-[[(4′-methoxy(1,1′-biphenyl)-4-yl]methoxy]-2-(4-morpholinyl)cyclopentyl]-5-heptenoate

A solution of the base derived from Intermediate 42 (0.5g) and triethylamine (0.28 ml) in dry $CH_3CN$ (30 ml) at −10° was treated with iso-butylchloroformate (0.26 ml) and 0.5h later with 4-hydroxybenzamide (0.685g) and dry pyridine (10 ml). After 1.5h the mixture was poured into pH 6.5 phosphate buffer (100 ml), extracted wth EA (2 × 100 ml), the combined extracts were washed with water (50 ml), dried and evaporated, and the residue purified by chromatography (HH) to give the *title compound* as a foam (0.49 g). I.R. ($CHBr_3$) 3520, 3400, 1755, 1675cm$^{-1}$.

Intermediate 44

[1α(Z),2β,3α,5α]-(±)-(Acetylthiomethyl) 7-5[[(1,1′-Biphenyl)-4-yl]methoxy]-3-hydroxy-2-(4-morpholinyl)cyclopentyl]-5-heptenoate

A mixture of Intermediate 33 (0.4 g), bromomethyl thioacetate (0.42 g) and anhydrous $K_2CO_3$ (0.23g) in dry DMF (9 ml) was stirred at 20° for 5.5h. The mixture was poured into pH 6.5 phosphate buffer (175 ml), extracted with EA (3 × 60 ml), the combined extracts dried and evaporated, and the residue purified by chromatography (G) to give the *title compound* as an oil (0.34 g). I.R. ($CHBr_3$) 3520, 1732, 1695cm$^{-1}$.

The preparation of Intermediate 45 is described in British Patent Specification 2075503A.

Intermediate 45

[1R-(exo,endo)]-(−)-2-Bromo-3-hydroxybicyclo[[3.2.0]heptan-6-one

Intermediate 46

[1R-(endo,anti)-(+)-5-Hydroxy-7-(1-piperidinyl)bicyclo[2.2.1]heptan-2-one

A solution of Intermediate 45 (5.25g) in acetone (50ml) containing piperidine (6.3 ml) was stirred at 20° for 2.5h in the dark. The mixture was poured into 8% $NaHCO_3$ solution (150ml) and extracted with $CH_2Cl_2$ (3 × 100ml). The combined extracts were dried and evaporated and the residue was purified by chromatography (C). The *title compound* was obtained as a solid (4.7 g). A portion was recrystallised from ER—PE to give material of m.p. 87—88°. $[\alpha]_D^{25.5}$=68.7°($CHCl_3$)

Intermediate 47

[1R-(endo,anti)]-(+)-5-[[(1,1′-Biphenyl)-4-yl]methoxy]-7-(1-piperidinyl)bicyclo[2.2.1]heptan-2-one

A mixture of Intermediate 46 (4.34 g), benzyltriethylammonium chloride (2g) and biphenylmethyl bromide (6.7g) in $CH_2Cl_2$ (100 ml) and 17N NaOH (60 ml) was stirred vigorously at 20° for 18h. The phases were separated and the aqueous phase, diluted with water (100 ml), was extracted with $CH_2Cl_2$ (3 × 100ml). The combined organic layers were washed with water (100 ml), dried and evaporated and the residue was purified by chromatography (SS through to S). The *title compound* was obtained as a solid (6.2 g). A portion was recrystallised from EA—PE (b.p. 60—80°) to give material of m.p. 108—110°. $[\alpha]_D^{22}$ = +25.45° ($CHCl_3$)

Intermediate 48

[1R-(endo,anti)]-(−)-6-[[(1,1′-Biphenyl)-4-yl]methoxy]-8-piperidinyl)-2-oxabicyclo[3.2.1.]octan-3-one

Peracetic acid (13ml; 6.12 M) was added dropwise to stirred solution of Intermediate 47 (5.8 g) in $CH_2Cl_2$ (150 ml) at 20°. The mixture was stirred for 20h, then diluted with water (250 ml). The phases were separated and the aqueous layer was extracted with $CH_2Cl_2$ (100ml). The combined organic layers were added to a cold (0°) saturated solution of $Na_2SO_3$ (150ml), then stirred vigorously at 20° for 1.5h. The mixture was diluted with isopropyl acetate (200ml) and the phases were separated. The aqueous layer was extracted with isopropyl acetate (2 × 100ml) and the combined organic layers were washed with 0.5N NaOH (100ml) and brine (150ml), then dried and evaporated. The residue was purified by chromatography

(EE) to give a solid which was recrystallised from ER—PE (b.p. 60—80°) to give the *title compound* as a colourless solid (2.3g), m.p. 129.5—130°. $[\alpha]_D^{22} = -26.5°$ ($CHCl_3$)

Intermediate 49

[1R-(1α,2β,3α,5α]-5-[[(1,1′-Biphenyl)-4-yl]methoxy]-3-hydroxy-2-(1-piperidinyl)cyclopentane acetaldehyde

A solution of Intermediate 48 (1.2g) in dry $CH_2Cl_2$ (20ml) was cooled (−78°) and stirred under nitrogen whilst a solution of DIBAL in hexane (5.25 ml; 1.43 M) was added dropwise. Methanol (20ml) was added dropwise at −70° after 0.75h and the cooling bath was removed. After stirring at 20° for 1h, the precipitate was filtered off and washed well with methanol. The combined filtrates were evaporated to give the *title compound* as a foam (1.2g). I.R. ($CHBr_3$) 3580, 3560, 2730, 1720cm$^{-1}$.

Intermediate 50

[1R-(1α,2β,3α,4α]-4-[[(1,1′-Biphenyl)-4-yl]methoxy]-3-(3-methoxy-2-propenyl)-2-(1-piperidinyl)cyclopentanol

(Methoxymethyl)triphenylphosphonium chloride (3.8g) was added over 10min to a cold (−5°) stirred solution of potassium *tert*-butoxide (1.27g) in dry THF (35ml). After 30 min, a solution of Intermediate 49 (1.18g) in THF (15ml) was added at 0° and stirring maintained for 30 min. The mixture was poured into 8% $NaHCO_3$ solution (150 ml) and extracted with EA (2 × 100ml). The combined extracts were dried and evaporated and the residue was purified by chromatography (FF) to give the *title compound* as a semi-solid (0.9g). I.R. ($CHBr_3$) 3580, 3500, 1650cm$^{-1}$.

Intermediate 51

[2R-(2α,3β,4β]-4-[[(1,1′-Biphenyl)-4-yl]methoxy]-3-(3-methoxy-2-propenyl)-2-(1-piperidinyl)cyclopentanone

A solution of Intermediate 50 (0.84 g) in $CH_2Cl_2$ (8 ml) was cooled (5°) whilst triethylamine (1.95ml) followed by pyridine-sulphur trioxide complex (1.27g) in DMSO (8ml) were added. After 1h at 5° the mixture was poured into pH 6 phosphate buffer (100ml) and extracted with ER (2 × 75ml). The combined extracts were washed with water (50ml) dried and evaporated and the residue was purified by chromatography (L) to give the *title compound* as an oil (0.725g). I.R. ($CHBr_3$) 1735, 1656cm$^{-1}$.

Intermediate 52

[1R-(1α,2β,3β,5α]-(+)-5-[[(1,1′-Biphenyl)-4-yl]methoxy]-3-hydroxy-2-(1-piperidinyl)cyclopentanepropanal

A stirred solution of Intermediate 51 (0.69g) in THF (15ml) was cooled (−10°) whilst lithium tri-sec-butylborohydride in THF (5ml; 1M) was added. After 1h at −10°, 2N HCl (20ml) was added, cautiously at first, and the mixture was stirred at 20° for 2h. The mixture was washed with ER (50ml) and then adjusted to pH 9 with 2N $Na_2CO_3$ and extracted with EA (4 × 50ml). The combined extracts were dried and evaporated and the residue was purified by chromatography (TT) to give the *title compound* as a foam (0.206g). $[\alpha]_D^{23} = +54.90°$ ($CHCl_3$). T.L.C. (TT) $R_f$0.13.

Intermediate 53

[1R-[1α(Z),2β,3β,5α]-(+)-Methyl 7-[5-[[(1,1′-Biphenyl)-4-yl]methoxy]- 3-hydroxy-2-(1-piperidinyl)cyclopentyl]-4-heptenoate

To a stirred solution of potassium t-butoxide (2.05g) in THF (80ml) was added (3-carboxypropyl)-triphenylphosphonium bromide (3.9g). After 0.5 h at 20° a solution of Intermediate 52 (0.463g) in THF (10ml) was added and stirring continued at 20° for 0.75h. Water (2ml) was added and the solvent removed *in vacuo*. The residue was taken into water (100ml), basified to pH 14 with 2N NaOH and washed with ER (3 × 60ml). The aqueous solution was adjusted to pH 6.5 with 2N HCl and extracted with $CH_2Cl_2$ (3 × 50ml). The combined extracts were concentrated, re-dissolved in 1:1 EA—$CH_2Cl_2$ (15ml) and then treated with an excess of an ethereal solution of diazomethane. Excess diazomethane was destroyed with acetic acid and the solution then diluted with EA (30ml) and washed with 2N $Na_2CO_3$ (40ml). The organic solution was dried and evaporated and the residue purified by chromatography (FF) to give the *title compound* as an oil (0.428g). $[\alpha]_D^{23} = +60.10°$ ($CHCl_3$). I.R. (Neat) 3600—3100 (br.), 1735cm$^{-1}$.

Intermediate 54

[1R-[1α(Z),2β,3β,5α]-(+)-7-[5-[[(1,1′-Biphenyl)-4-yl]methoxy]-3-hydroxy-2-(1-piperidinyl)cyclopentyl]-4-heptenoic acid, hydrochloride

Intermediate 53 (10.38g) was stirred with 74 OP ethanol (60 ml) and 5N NaOH (30ml) at 20° for 16h. The solution was diluted with water (400ml) and then extracted with ER (2 × 150ml). The aqueous phase was adjusted to pH 6 with 2N HCl and extracted with $CH_2Cl_2$ (3 × 200ml). Evaporation of the combined extracts gave a foam (9.45g), the majority (9.3g) of which was taken up into $CH_2Cl_2$ (50ml) and treated with an excess of an ethereal solution of hydrogen chloride. Evaporation *in vacuo* and trituration of the residue with ER (4 × 75ml) gave the *title compound* as a powder (9.28g). Crystallisation of a sample from EA-methanol gave material of m.p. 124—126°. $[\alpha]_D^{25} = +63.1°$ ($CHCl_3$)

The preparation of Intermediate 55 is described in British Patent Specification 2075503A.

Intermediate 55

(endo,anti)-(±)-5-Hydroxy-7-(1-piperidinyl)bicyclo[2.2.1]heptan-2-one, hydrochloride

Intermediate 56

(endo,anti)-(±)-5-[[4'-Methoxy(1,1'-Biphenyl)-4-yl]methoxy]-7-(1-piperidinyl)bicyclo[2.2.1]heptan-2-one

A mixture of Intermediate 55 (6.64 g), benzyltriethylammonium chloride (2g) and 4-(bromomethyl)-4'-methoxy(1,1'-biphenyl) (9.73g) in $CH_2Cl_2$ (100ml) and 17N NaOH (70ml) was vigorously stirred at 20° for 16h. The mixture was poured into water (140ml), the phases separated and the aqueous layer was extracted with $CH_2Cl_2$ (100ml). The combined organic layers were dried and evaporated and the residue was triturated with PE (b.p. 60—80°) (50ml) to give a solid which was recrystallised from 5:2 EA—PE (b.p. 60—80°) to give the *title compound* as a solid (6.63g), m.p. 112—115°.

Intermediate 57

(endo,anti)-(±)-6-[[4'-Methoxy(1,1'-biphenyl)-4-yl]methoxy]-8-(1-piperidinyl)-2-oxobicyclo[3.2.1]octan-3-one

Peracetic acid (11.2ml; 6.12M) was added slowly to a cooled (5°) stirred solution of Intermediate 56 (6.1g) in $CH_2Cl_2$ (25ml). The mixture was stirred at 20° for 64h then added slowly to a cooled (5°) stirred mixture of saturated $Na_2SO_3$ (70ml) and water (30ml). After 1h, isopropyl acetate (50ml) was added and the layers were separated. The aqueous layer was extracted with isopropyl acetate (2 × 50ml) and the combined organic layers were washed with 1N NaOH (100ml) and dried and evaporated. The residue was purified by chromatography (UU) to give an oil which was triturated with ER to give the *title compound* as a solid (2g), m.p. 105—106°.

Intermediate 58

(1α,2β,3α,5α)-(±)-3-Hydroxy-5-[[4'-methoxy(1,1'-biphenyl)-4-yl]methoxy]-2-(1-piperidinyl)cyclopentane acetaldehyde

DIBAL in hexane (7.9ml); 1.43M) was added over 0.5h to a cold (−70°), stirred solution of Intermediate 57 (1.9g) in $CH_2Cl_2$ (15ml). After 1.5h methanol (15ml) was added dropwise and the mixture was stirred at 20° for 2h. The preparation was filtered off and the solid was washed with methanol. The combined filtrates were evporated and the residue was dissolved in $CH_2Cl_2$ (50ml), dried and the solvent was removed to give the *title compound* as a foam (1.89g).

I.R. ($CHBr_3$) 3580, 3535, 2730, 1710cm$^{-1}$.

Intermediate 59

(1α,2β,3α,4α)-(±)-4-[[4'-Methoxy(1,1'-biphenyl)-4-yl]methoxy]-3-(3-methoxy-2-propenyl)-2-(1-piperidinyl)cyclopentanol

(Methoxymethyl)triphenylphosphonium chloride (6.17g) was added over 10min to a cold (0°) solution of potassium *tert*-butoxide (2.02g) in dry THF (35ml). After 15 min, a solution of Intermediate 58 (1.86g) in THF (10ml) was added dropwise and stirring was maintained for 1.5h. The mixture was poured into pH 6 phosphate buffer (100ml) and extracted with EA (2 × 50 ml). The combined extracts were dried and evaporated and the residue was purified by chromatography (FF) to give the *title compound* as a semi-solid (1.29g).

I.R. ($CHBr_3$) 3520, 3330, 1653cm$^{-1}$.

Intermediate 60

(2α,3β,4β)-(±)-4-[[4'-Methoxy(1,1'-biphenyl)-4-yl]methoxy]-3-(3-methoxy-2-propenyl)-2-piperidinyl) cyclopentanone

A solution of Intermediate 59 (0.4g) in $CH_2Cl_2$ (4ml) was cooled (0°) whilst triethylamine (0.95ml) followed by pyridine-sulphur trioxide complex (0.65g) in DMSO (4ml) were added. After 1h at 0° the mixture was poured into pH 6.5 phosphate buffer (50ml) and extracted with ER (2 × 50ml). The combined extracts were washed with brine 2 × 25ml) dried and evaporated and the residue was purified by chromatography (S) to give the *title compound* as an oil.

I.R. (Neat) 1740, 1655cm$^{-1}$.

Intermediate 61

(1α,2β,3β,5α)-(±)-3-Hydroxy-5-[[4'-methoxy(1,1'-biphenyl)-4-yl]methoxy]-2-(1-piperidinyl)cyclopentanepropanol

DIBAL (1.43M in hexane, 14ml) was added over 10 min under nitrogen to an ice cooled solution of 2,6-di-t-butyl-p-cresol (8.8g) in dry toluene (100ml). After 1h, the solution was cooled to −45° and a solution of Intermediate 60 (0.62g) in toluene (20ml) was added over 3min. The temperature was allowed to rise to −10° over 1h, then 2N HCl (40ml) was added and the mixture stirred at room temperature for 1.5h. The mixture was diluted with ER (100ml) and the organic layer extracted with 1N $H_2SO_4$ (30ml). The combined aqueous solutions were washed with ER (100ml), basified with solid $NaHCO_3$ and the product extracted into $CH_2Cl_2$ (3 × 80ml). The combined extracts were dried ($Na_2SO_4$) and concentrated, and the residue purified by chromatography (TT) to give the *title compound* as an oil (0.43g).

I.R. (Neat) 3400(br.), 1720 cm$^{-1}$.

Intermediate 62

[1α,(Z),2β,3β,5α]-(±)-7-[3-Hydroxy-5-[[4'-methoxy(1,1'-biphenyl)-4-yl]methoxy]-2-(1-piperidinyl)cyclopentyl]-4-heptenoic acid, compound with ethyl acetate and dichloromethane (20:3:2)

(3-Carboxypropyl)triphenylphosphonium bromide (1.55g) was added under nitrogen to a stirred solution of potassium t-butoxide (0.775g) in dry THF (25ml). After 40min a solution of Intermediate 61 (0.43g) in THF (7ml) was added and the mixture stirred for 35 min at room temperature. Water (80ml) followed by 2N NaOH (5ml) were added and the mixture extracted with ER (2 × 100ml). The aqueous solution was neutralised with 2N $H_2SO_4$, treated with pH 6.5 phosphate buffer (10%, 25ml) and extracted with EA (2 × 70ml). The combined extracts were washed with phosphate buffer (3 × 50ml), dried ($Na_2SO_4$) and evaporated *in vacuo* to give the *title compound* as a foam (0.3g).

I.R. ($CHBR_3$) 3200—2300(br.), 1720(br.) $cm^{-1}$.

Intermediate 63

[1R-[1α(Z),2β,3α,5α]-(±)-7-[5-[[(1-1'-Biphenyl)-4-yl]methoxy]-3-hydroxy-2-(1-piperidinyl)cyclopentyl]-5-heptenoic acid

To a stirred solution of potassium-t-butoxide (5.9g) in dry THF (70ml) was added (4-carboxybutyl)triphenylphosphonium bromide (11.08g) with stirring maintained at 20° under dry nitrogen for 0.5h. A solution of Intermediate 49 (3.0g) in dry THF (30ml) was added and stirring continued at 20° for 50min, whereupon water (15ml) was added and excess solvent removed *in vacuo*. After dilution with water (20ml) the solution was basified with 2N NaOH (30ml) and extracted with ER (3 × 100ml). The aqueous phase was adjusted to pH 7 with 2N HCl (35ml) and extracted with $CH_2Cl_2$ (2 × 100ml). The solution was then adjusted to pH 6 and extracted with $CH_2Cl_2$ (2 × 100ml) and finally to pH 5.5 and extracted with $CH_2Cl_2$ (100ml). The combined extracts were dried and evaporated to afford the crude *title compound* as a foam (3.71g) which was purified via its methyl ester as described below. To a stirred solution of the foam (0.496g) in methanol (10ml) at 20° was added concentrated $H_2SO_4$ (0.2ml) and the mixture stirred for 2.75h. The solution was then poured into 8% aqueous $NaHCO_3$ (20ml) and extracted with $CH_2Cl_2$ (3 × 20ml). The combined extracts were dried and evaporated, and the residue purified by chromatography (FF) to give an oil (0.438g). To a solution of this oil (0.4g) in methanol (2ml) was added 5N NaOH (1.5ml) and the mixture stirred at 20° for 2.3h. After dilution with water (20ml) the solution was extracted with ER (2 × 20ml). The aqueous solution was adjusted to pH 6 with 2N HCl (2ml) followed by pH 6 buffer solution (20ml) and extracted with $CH_2Cl_2$ (3 × 20ml). The combined extracts were dried and evaporated to afford the pure *title compound* as a foam (0.376g).

I.R. ($CHBr_3$) 3500, 1710(br), 1520 $cm^{-1}$.

$[\alpha]_D^{26}$ = +30.5° ($CHCl_3$).

Intermediate 64

[1R-1α(Z),2β,5α]-Triphenylmethyl 7-[5-[[(1,1'-Biphenyl)-4-yl]methoxy]-3-oxo-2-(1-piperidinyl)cyclopentyl]-5-heptenoate

To a solution of Intermediate 63 (0.503g) in $CH_2Cl_2$ (5ml) at 4° was added trityl chloride (0.391g) followed by triethylamine (0.22ml) and the mixture stirred in an ice-bath for 0.5h. More triethylamine (1.03ml) was added followed by a solution of pyridine-sulphur trioxide complex (0.67g) in dry DMSO (5ml) and stirring maintained at 0—10° for 3h. The mixture was poured into pH 6 phosphate buffer solution (100ml) then extracted with ER (100ml, 2 × 50ml). The combined extracts were washed with water (50ml), dried and evaporated, and the residue purified by chromatography (L) to give the *title compound* as a foam (0.462g).

I.R. ($CHBr_3$) 1740 $cm^{-1}$.

Intermediate 65

[1R-[1α(Z),2β,3β,5α]-(+)-7-[5-[[(1,1'-Biphenyl)-4-yl]methoxy]-3-hydroxy-2-(1-piperidinyl)cyclopentyl]-5-heptenoic acid, compound with dicyclohexylamine (1:1)

DIBAL in hexane (1M, 32ml) was added dropwise at 0° over 5min to a solution of 2,6-di-t-butyl-4-methylphenol (13.47g) in dry toluene (75ml) under dry nitrogen and stirring maintained at 0° for 1h. The solution was then cooled to −65° and a solution of Intermediate 64 (1.39g) in dry toluene (25ml) added dropwise. The mixture was stirred at −70° for 0.75h, allowed to warm to −20° over 0.5h and stored at that temperature for 14h. After addition of 2N HCl (100 ml) the mixture was stirred at 20° for 2h, then poured into water (100ml) and ER (100ml). The aqueous phase was separated, washed with ER (100ml), then adjusted to pH 6 with 5N NaOH (35ml) followed by pH 6 phosphate buffer solution (50ml). This mixture was then extracted with $CH_2Cl_2$ (4 × 100ml) and the combined extracts dried and concentrated to yield a foam (1.1g). A portion (0.22g) was dissolved in $CH_2Cl_2$ (2ml) and treated with dicyclohexylamine (0.09ml). After evaporation *in vacuo* the residue was triturated with solvent mixture (L) whereupon the *title compound* (0.23g) crystallised, m.p. 101—103°. $[\alpha]_D^{24}$ = +27.3° ($CHCl_3$).

The preparation of Intermediate 66 is described in British Patent Specification 2075503A.

Intermediate 66

[1α(Z),2β,5α]-(±)-7-[5-[[4'-Methyl(1,1'-biphenyl)-4-yl]methoxy]-2-(4-morpholinyl)-3-oxocyclopentyl]-4-heptenoic acid.

Intermediate 67

[1α(Z),2β,3β,5α]-(±)-7-[3-Hydroxy-5-[[4'-methyl(1,1'-biphenyl)-4-yl]methoxy]-2-(4-morpholinyl)cyclopentyl]-4-heptenoic acid

A solution of Intermediate 66 (0.105g) in dry THF (25ml) was added over 5min under nitrogen to a stirred solution of lithium tri-sec-butylborohydride (1M in THF; 3ml) at −30°. After 2h the solution was allowed to rise to ambient temperature and then poured into 2N $H_2SO_4$ (15ml) and extracted with ER (2 × 30ml). The aqueous phase was adjusted to pH 6 and extracted with EA (3 × 30ml). The combined extracts were washed with pH 6.5 phosphate buffer solution (15ml), dried and concentrated. The residue was purified by chromatography (FF) to give the *title compound* as a foam (0.063g).

I.R. ($CHBr_3$) 3500, 3400—2300, 1730, 1710cm$^{-1}$.

Example 1

*a) [1α[Z],2β,5α]-(±)-(Methylthio)methyl 7-[5-[[(1',1-Biphenyl)-4-yl]methoxy]-2-(4-morpholinyl)-3-oxocyclopentyl]-4-heptenoate*

Chloromethyl methyl sulphide (0.11 ml) was added to a stirred solution of Intermediate 1 (0.4 g) and dicyclohexylamine (0.18 ml) in dry $CH_3CN$ (6 ml). After 24h the suspension was poured into saturated $NH_4Cl$ solution (40 ml) and extracted with EA (3 × 30 ml). The combined extracts were dried, filtered, and evaporated to afford a mobile oil. Purification by chromatography (H) gave the *title compound* as a solid (0.178 g), m.p. 36—38°.

I.R. (Neat) 1740 cm$^{-1}$.

The following compound was prepared by a similar procedure:

*b) [1α[Z],2β,5α] - (±) - (Methylthio)methyl 7 - [5 - [[4' - Methoxy(1,1' - biphenyl) - 4 - yl]methoxy] - 3 - oxo - 2 - (4 - thiomorpholinyl)cyclopentyl) - 4 - heptenoate,* S-dioxide, m.p. 100—101.5° from Intermediate 2. Purification initially by chromatography (C) and then by trituration with ER.

I.R. ($CHBr_3$) 1740cm$^{-1}$.

Example 2

*a) [1α(Z),2β,5α]-(±)-4-(Acetylamino)phenyl 7-[5-[[(1,1'-Biphenyl)-4-yl]methoxy]-2-(4-morpholinyl)-3-oxocyclopentyl]-4-heptenate*

Method 1

Iso-butylchloroformate (0.20 ml) was added to a cold (−10°) solution of Intermediate 1 (0.24 g) and triethylamine (0.28 ml) in high purity acetone (5 ml). The mixture was stirred for 10 min whereupon 4-(acetylamino) phenol (0.38 g) was added and stirring maintained for a further 2.5h. Excess solvent was removed in vacuo, the residue treated with pH 6.5 buffer solution (30 ml) and extracted with EA (3 × 25 ml). The combined extracts were dried, filtered and evaporated to afford a crude product which was dissolved in ER (50 ml) and washed with 8% $NaHCO_3$ solution (2 × 20 ml) to remove excess phenol. The organic layer was dried, filtered and evaporated, and the residue purified by chromatography (I) to give the *title compound* as a foam (0.235g).

I.R. ($CHBr_3$) 3420, 1755(sh), 1740, 1685, 1510cm$^{-1}$.

T.L.C. (I) Rf 0.24.

Method 2

To a solution of the compound of Example 21 (0.045 g) in $CH_2Cl_2$ (1 ml) at 20° was added triethylamine (0.08 ml) and pyridine sulphur trioxide complex (0.1g) in DMSO (1 ml). After 2h the solution was poured into pH 6 phosphate buffer solution (25 ml), extracted with ER (2 × 25 ml), the combined extracts washed with $H_2O$ (20 ml), dried and evaporated. The residue was purified by chromatography (F) to give the *title compound* (0.028 g). I.R. ($CHBr_3$) 3420, 1755(sh), 1740, 1690, 1510cm$^{-1}$.

The following compounds were prepared according to the procedure described for Method 1:

*b) [1α(Z),2β,5α] - (±) - 2 - Methoxyethyl 7 - [5[[(1,1' - Biphenyl) - 4 - yl]methoxy] - 2 - (4 - morpholinyl) - 3 - oxocyclopentyl] - 4 - heptenoate* from Intermediate 1 (0.5 g) and 2-methoxyethanol (0.43 ml). Purification by chromatography (N) gave an oil (0.39 g).

I.R. (Neat) 1740cm$^{-1}$.

| | | | |
|---|---|---|---|
| Analysis Found: | C, 71.8; | H, 7.9; | N, 2.9; |
| $C_{32}H_{41}NO_6$ requires: | C, 71.7; | H, 7.7; | N, 2.6% |

*c) [1α(Z),2β,5α] - (±) - 2 - (4 - Morpholinyl)ethyl 7 [2 - 4 - Morpholinyl) - 3 - oxo - 5 - [4 - phenylmethyl)-phenylmethoxy]cyclopentyl] - 4 - heptenoate* from the free acid derived from Intermediate 3 (0.152 g) and N-2-hydroxyethyl morpholine (0.44 g) in dry $CH_3CN$ (6 ml). Purification by chromatography (G) gave an oil (0.071 g).

I.R. ($CHBr_3$) 1735cm$^{-1}$.

| | | | |
|---|---|---|---|
| Analysis Found: | C, 71; | H, 8.0; | N, 4.5; |
| $C_{36}H_{48}N_2O_6$ requires: | C, 71.5; | H, 8.0; | N, 4.6% |

*d) [1α(Z),2β,5α] - (±) - 2 - Dimethylamino)ethyl 7 - [5 - [[(1,1' - Biphenyl) - 4 - yl]methoxy] - 2 - (4 - morpholinyl) - 3 - oxocyclopentyl] - 4 - heptenoate* from Intermediate 1 (0.35 g) and N,N-dimethylethanolamine (0.22 ml). Purification by chromatography (o) gave an oil (0.366 g).

I.R. (Neat) $1740cm^{-1}$.

| | | | |
|---|---|---|---|
| Analysis Found: | C, 72.2; | H, 7.7; | N, 5.2; |
| $C_{33}H_{44}N_2O_5$ requires: | C, 72.2; | H, 8.1; | N, 5.1% |

*e) [1α(Z),2β,5α] - (±) - 2 - (Acetylamino)phenyl 7 - [5 - [[(1,1' - Biphenyl) - 4 - yl]methoxy] - 2 - (4 - morpholinyl) - 3 - oxocyclopentyl] - 4 - heptenoate* m.p. 82—84° from Intermediate 1 (0.477g) in dry $CH_3CN$ (30 ml) and 2-(acetylamino)phenol (0.755g) in dry pyridine (10 ml). Purification initially by chromatography (P) and then recrystallisation from EA/PE (b.p. 60—80°) gave 0.102g of the *title compound.*

I.R. ($CHBr_3$) 3440, 1760, 1740, $1695cm^{-1}$.

*f) [1α(Z),2β,5α] - (±) - 3 - (Acetylamino)phenyl 7 - [5 - [[(1,1' - Biphenyl) - 4 - yl]methoxy] - 2 - (4 - morpholinyl) - 3 - oxocyclopentyl]-4-heptenoate* from Intermediate 1 (0.477g) in dry $CH_3CN$ (30 ml) and 3-acetylamino)phenol (0.755g) in dry pyridine (10 ml). Purification by chromatography (P) gave an oil (0.533g).

I.R. ($CHBr_3$) 3470, 1738, 1689, $1520cm^{-1}$.

| | | | |
|---|---|---|---|
| Analysis Found: | C, 72.3; | H, 6.6; | N, 4.4; |
| $C_{37}H_{42}N_2O_6$ requires: | C, 72.8; | H, 6.9; | N, 4.6% |

*g) [1α(Z),2β,5α] - (±) - S - 4 - (Acetylamino)phenyl 7 - [5 - [[(1,1' - Biphenyl) - 4 - yl]methoxy] - 2 - (4-morpholinyl) - 3 - oxocyclopentyl] - 4 - heptenethioate* from Intermediate 1 (0.275 g) and 4-(acetylamino)thiophenol (0.484 g). Purification twice by chromatography, firstly with (Q) and then (E) afforded a foam (0.277 g).

I.R. ($CHBr_3$) 3420, 1732, $1690cm^{-1}$.

T.L.C. (Q) Rf 0.22.

*h) [1α(Z),2β,5α(E)] - (±) - 1,3-Bis(dimethylamino) - 2 - propyl 7 - [2 - (4 - Morpholinyl) - 3 - oxo - 5 - [(3-phenyl - 2 - propenyl)oxyl]cyclopentyl] - 4 - heptenoate* from Intermediate 4 (0.4g) and 1,3-Bis(dimethylamino)-2-propanol (0.45 ml). Purification by chromatography (R) gave an oil (0.13g).

I.R. (Neat) 1740, $968cm^{-1}$.

| | | | |
|---|---|---|---|
| Analysis Found: | C, 69.1; | H, 9.2; | N, 75.; |
| $C_{32}H_{49}N_3O_5$ requires: | C, 69.2; | H, 8.9; | N, 7.6% |

*i) [1α(Z),2β,5α] - (±) - 2,2,2 - Trichloroethyl 7 - [5 - [[(1,1' - Biphenyl) - 4 - yl]methoxy - 2 - (4 - morpholinyl) - 3 - oxocyclopentyl] - 4 - heptenoate* m.p. 86.5—88° from Intermediate 1 (0.8g) and 2,2,2-trichloroethanol (0.76 ml). Purification initially by chromatography (S) and then by trituration with ER gave a solid (0.22 g).

I.R. ($CHBr_3$) $1740cm^{-1}$

| | | | |
|---|---|---|---|
| Analysis Found: | C, 61.3; | H, 5.9; | N, 2.4; |
| $C_{31}H_{36}Cl_3NO_5$ requires: | C, 61.0; | H, 6.0; | N, 2.3% |

*j) [1α(Z),2β,5α] - (±) - 3,3,5 - Trimethylcyclohexyl 7 - [5 - [[(1,1' - Biphenyl) - 4 - yl]methoxy] - 2 - (4 - morpholinyl) - 3 - oxocyclopentyl] - 4 - heptenoate* from Intermediate 1 (1.09 g) in acetone (12 ml) and 3,3,5-trimethylcyclohexanol (1.028 g) in dry pyridine (8 ml). Purification by chromatography (X) gave an oil (0.114 g).

I.R. (Neat) $1740cm^{-1}$.

T.L.C. (X) Rf 0.1.

*k) [1α(Z),2β,5α] - (±) - S - 2 - (Methoxythio)ethyl 7 - [5 - [[(1,1' - Biphenyl) - 4 - yl]methoxy] - 2 - (4 - morpholinyl) - 3 - oxocyclopentyl] - 4 - heptenethioate* m.p. 35—37° from Intermediate 1 (0.66 g) in dry $CH_3CN$ (40 ml) and 2-(methylthio)ethane thiol (0.68 g) in dry pyridine (10 ml). Purification initially by chromatography (C) and then by trituration with ice-cold PE (b.p. 40—60°) gave a solid (0.251 g).

I.R. ($CHBr_3$) 1740, $1690cm^{-1}$.

*l) [1α(Z),2β,5α] - (±) - 2 - Propenyl 7 - [5 - [[(1,1' - Biphenyl) - 4 - yl]methoxy] - 2 - (4 - morpholinyl) - 3 - oxocyclopentyl] - 4 - heptenoate* from Intermediate 1 (0.57 g) and 2-propen-1-ol (1.8 ml). Purification by chromatography (K) gave an oil (0.17 g).

I.R. (Neat) $1735cm^{-1}$.

| | | | |
|---|---|---|---|
| Analysis Found: | C, 74.2; | H, 7.5; | N, 2.7; |
| $C_{32}H_{39}NO_5$ requires: | C, 74.25; | H, 7.6; | N, 2.7% |

*m) [1α(Z),2β,5α] - (±) - 2 - Furanylmethyl 7 - [5 - [[(1,1' - Biphenyl) - 4 - yl]methoxy] - 2 - (4 - morpholinyl) - 3 - oxocyclopentyl] - 4 - heptenoate, compound with water (1:0.5)* from Intermediate 1 (0.5 g) and 2-furanmethanol (0.616g). Purification by chromatography (V) gave an oil (0.442g).

I.R. (Neat) 3665, 1739cm$^{-1}$.

| | | | |
|---|---|---|---|
| Analysis Found: | C, 72.3; | H, 7 .1; | N, 2.4; |
| $C_{34}H_{39}NO_6 \cdot 0.5H_2O$ requires: | C, 72.0; | H, 7.0; | N, 2.5% |

## Example 3

*a) [1α(Z),2β,5α] - (±) - (Acetyloxy)methyl 7 - [5 - [[(1,1' - Biphenyl) - 4 - yl]methoxy] - 2 - (4 - morpholinyl) - 3 - oxocyclopentyl] - 4 - heptenoate*

Method 1

Bromomethyl acetate in (0.12 g) was added to a solution of Intermediate 1 (0.25 g) and triethylamine (0.146 ml) in acetone (5 ml) and the mixture stirred at room temperature for 2.5h. The mixture was poured into water (100 ml) and extracted with ER (4 × 40 ml). The organic layers were dried and evaporated to give an oil (0.287 g). Purification by chromatography (J) gave the *title compound* as a solid (0.175 g), m.p. 35—37°.

I.R. ($CHBr_3$) 1760, 1740cm$^{-1}$.

Method 2

To a cold (0°), stirred solution of Intermediate 21a (0.19g) in acetone (10 ml) was added Jones reagent (2.6M, 0.17 ml). The mixture was kept at 0° for 6h, whereupon IPA (0.5 ml) was added, followed 10 min later by 8% $NaHCO_3$ solution (35 ml). The resulting suspension was extracted with EA (2 × 40 ml). The dried extracts were evaporated and the residue purified by chromatography (K) to give the *title compound* (0.056g).

I.R. (CHBr) 1760, 1740cm$^{-1}$.

Method 3

To a solution of the compound of Example 20 (0.054 g) in $CH_2Cl_2$ (0.75 ml) was added triethylamine (0.15 ml), followed by pyridine sulphur trioxide complex (0.13 g) in DMSO (1 ml). The mixture was stirred at 21° for 1h, poured into pH 6 phosphate buffer solution (30 ml) and extracted with ER (2 × 25 ml). The combined extracts were dried and evaporated and the residue purified by chromatography (C) to give the *title compound* (0.04 g).

I.R. ($CHBr_3$) 1760, 1740cm$^{-1}$.

Method 4

Lindlar catalyst (0.007 g) was hydrogenated at 18° and atmospheric pressure in EA (4 ml) containing quinoline (0.003 g) until uptake ceased. A solution of Intermediate 21b (0.04 g) in EA (4 ml) was added and hydrogenation continued for 54 min (uptake ceased). The catalyst was filtered off, the filtrate evaporated and the residue purified by chromatography (L) to give the *title compound* (0.039 g).

I.R. ($CHBr_3$) 1760, 1740cm$^{-1}$.

The following compounds were prepared using a similar manner to Method 1.

*b) 1α(Z),2β,5α] - (±) - 1 - (Acetyloxy)ethyl 7 - [5 - [[(1,1' - Biphenyl) - 4 - yl]methoxy] - 2 - (4 - morpholinyl) - 3 - oxocyclopentyl] - 4 - heptenoate, compound with diethyl ether (10:1)* from Intermediate 1 (0.435g) and 1-bromoethyl acetate (0.456g). Purification by chromatography (T) gave an oil (0.234 g).

I.R. (Neat) 1760, 1745cm$^{-1}$.

| | | | |
|---|---|---|---|
| Analysis Found: | C, 69.8; | H, 7.6; | N, 2.4; |
| $C_{33}H_{41}NO_7.0.IC_4H_{10}O$ requires: | C, 70.2; | H, 7.4; | N, 2.4% |

*c) 1α(Z),2β,5α] - (±) - 1 - (Acetyloxy)methyl 7 - [5 - [[(1,1' - Biphenyl) - 4 - yl]methoxy] - 3 - oxo - 2 - (4 - thiomorpholinyl)cyclopentyl] - 4 - heptenoate* from Intermediate 5 (0.251 g) and bromomethyl acetate (0.155g). Purification by chromatography (U) afforded an oil (0.211g).

I.R. (Neat) 1765, 1745cm$^{-1}$.

| | | | |
|---|---|---|---|
| Analysis Found: | C, 67.9; | H, 6.9; | N, 2.45; |
| $C_{32}H_{39}NO_6S$ requires: | C, 67.9; | H, 6.95; | N, 2.5% |

## Example 4

[1α(Z),2β,5α]-(±)-Triphenylmethyl 7-[5-[[(1,1'-Biphenyl)-4-yl)methoxy]-2-(4-morpholinyl)-3-oxocyclopentyl]-4-heptenoate

From Intermediate 1 (1.5 g) and triphenylmethyl chloride (1.315 g) in $CH_2Cl_2$ (9 ml) according to the method described for Example 3a (Method 1). Purification by chromatography (C) gave a foam (1.843 g).

I.R. ($CHBr_3$) 1740cm$^{-1}$.

| | | | |
|---|---|---|---|
| Analysis Found: | C, 80.2; | H, 6.9; | N, 2.1; |
| $C_{48}H_{49}NO_5$ requires: | C, 80.1; | H, 6.9; | N, 1.95% |

Example 5

[1α(Z),2β,5α]-(±)-(Ethoxycarbonyl)methyl 7-[5-[[(1,1′-Biphenyl-4-yl)methoxy]-2-(4-morpholinyl)-3-oxocyclopentyl]-4-heptenoate

Method 1

From Intermediate 1 (0.3g) and ethyl bromoacetate (0.222 ml) in dry $CH_3CN$ (5 ml) using the procedure described for Example 3a, Method 1. Purification by chromatography (V) gave an oil (0.329 g).

I.R. (neat) 1750 (sh), 1740cm$^{-1}$.

| | | | |
|---|---|---|---|
| Analysis Found: | C, 70.3; | H, 7.7; | N, 2.1; |
| $C_{33}H_{41}NO_7$ requires: | C, 70.3; | H, 7.3; | N, 2.5% |

Method 2

A solution of Intermediate 1 (0.245 g) and ethyl diazoacetate (0.065 ml) in dioxan (5 ml) containing copper (II) chloride (0.003g) was stirred for 18h at room temperature. The reaction mixture was poured into pH 6 phosphate buffer (40 ml) and extracted with $CH_2Cl_2$ (3 × 30 ml). The combined extracts were dried and evaporated, and the residue purified by chromatography (H) to give the *title compound* (0.119g).

I.R. (Neat) 1750 (sh) 1740cm$^{-1}$.

Example 6

*a) [1α(Z),2β,5α] - (±) - 2 - Oxo - 2 - phenylethyl 7 - [5 - [[(1,1′ - Biphenyl - 4 - yl)methoxy] - 2 - (4 - morpholinyl) - 3 - oxocyclopentyl] - 4 - heptenoate* α-Bromoacetophenone (0.252g) was added to a stirred solution of Intermediate 1 (0.3g) and diisopropylethylamine (0.24 ml) in dry $CH_3CN$ (8 ml). Afer 1h the solution was poured into pH 6.5 buffer (30 ml) and extracted with EA (3 × 25 ml). The combined extracts were dried, filtered and evaporated, and the residue purified by chromatography (N) to afford the *title compound* as an oil (0.353 g).

I.R. (Neat) 1740, 1700cm$^{-1}$.

| | | | |
|---|---|---|---|
| Analysis Found: | C, 74.1; | H, 7.3; | N, 2.1; |
| $C_{37}H_{41}NO_6$ requires: | C, 74.6; | H, 6.9; | N, 2.4% |

The following compound was prepared in a similar manner:

*b) [1α(Z),2β,5α] - (±) - Tetrahydro - 2 - oxofuran - 3 - yl 7 - [5 - [[(1,1′ - Biphenyl) - 4 - yl]methoxy] - 2 - (4 - morpholinyl) - 3 - oxocyclopentyl] - 4 - heptenoate* from Intermediate 1 (0.3g) and 3-bromo-dihydro-2(3H)-furanone (0.24 ml). Purification by chromatography (C) gave an oil (0.272 g).

I.R. (Neat) 1790, 1740cm$^{-1}$.

T.L.C. (C) Rf 0.24.

Example 7

[1α(Z),2β,5α]-(±)-Tetrahydro-2-oxofuran-2-yl 7-[5-[[(1,1′-Biphenyl)-4-yl)methoxy]-2-(4-morpholinyl)-3-oxocyclopentyl]-4-heptenoate

5-Chloro-dihydro-2(3H)-furanone (0.152g) was added to a mixture of Intermediate 1 (0.2g) and trioctylpropyl ammonium bromide (0.4g) in diisopropylethylamine (0.44 ml) and dry $CH_3CN$ (3 ml). The mixture was stirred at room temperature for 2h, poured into aqueous pH 6.5 phosphate buffer (65 ml) and then extracted with EA (3 × 20 ml). The dried extracts were evaporated and the residue purified by chromatography (W) to give the *title compound* as an oil (0.16g).

I.R. (Neat) 1800, 1750cm$^{-1}$.

T.L.C. (C) Rf 0.23.

Example 8

[1α(Z),2β,5α]-(±)-(Acetylthio)methyl 7-[5-[[(1,1′-Biphenyl)-4-yl]methoxy]-2-(4-morpholinyl)-3-oxocyclopentyl]-4-heptenoate

Bromomethylthioacetate (0.377 g) was added to a mixture of Intermediate 1 (0.426 g) and anhydrous $K_2CO_3$ (0.246 g) in dry DMF (8 ml) and the mixture then stirred for 3h. The mixture was poured into aqueous pH 6.5 phosphate buffer (170 ml) and extracted with EA (3 × 60 ml). The combined organic layers were dried and evaporated, and the residue purified by chromatography (C) to give the *title compound* as an oil (0.269 g).

I.R. (Neat) 1740, 1708cm$^{-1}$.

T.L.C. (C) Rf 0.44.

Example 9

*a) [1α(Z),2β,5α]-(±)-(Methylthio)methyl 7-[5-[[4′-Methoxy(1,1′-biphenyl)-4-yl]methoxy]-2-(4-morpholinyl)-3-oxocyclopentyl]-4-heptenoate*

Pyridinium trifluoroacetate (0.375 g) was added to a stirred mixture of Intermediate 6 (0.5 g) and dicyclohexylcarbodiimide (1 g) in dry DMSO (5 ml), under nitrogen, with water bath cooling. After 5.5 h, the mixture was diluted with $H_2O$ (130 ml) and extracted with $CH_2Cl_2$ (3 × 50 ml). The organic solution was dried ($Na_2SO_4$) and evaporated *in vacuo* to leave a semi-solid 1.5 g) which was triturated with ER (4 × 5 ml), the solid filtered off and the ER solution evaporated to give an oil (1 g). Chromatography (H) gave, after trituration with ER, the *title compound* (0.31 g) as a solid m.p. 68—72°.

I.R. ($CHBr_3$) 1740cm$^{-1}$.

The following compounds were similarly prepared:

*b) [1α(Z),2β,5α]-(±) - (Methylthio)methyl 7 - [5 - [[4' - Methyl(1,1' - biphenyl) - 4 - yl]methoxy] - 2 - (4-morpholinyl) - 3 - oxocyclopentyl] - 4 - heptenoate,* m.p. 56.5—59.5° from Intermediate 7 (0.3 g). Purification by chromatography (N) gave an oil (0.14 g) which crystallised on standing.

I.R. ($CHBR_3$) 1735cm$^{-1}$.

*c)[1α(Z),2β,5α(E)] - (±) - (Methylthio)methyl 7 - [2 - (4 - Morpholinyl) - 3 - oxo - 5 - [(3 - phenyl - 2 - propenyl)oxy]cyclopentyl] - 4 - heptenoate,* from Intermediate 8 (0.257 g). Purification by chromatography (T) gave an oil (0.136 g).

I.R. (Neat) 1735, 970cm$^{-1}$

| | |
|---|---|
| Analysis Found: | C, 66.3; H, 7.6; N, 2.9; |
| $C_{27}H_{37}NO_5S$ requires: | C, 66.5; H, 7.7; N, 2.9% |

Example 10

[1α(Z),2β,5α]-(±)-(Methylthio)methyl 7-[5-[[1,1'-Biphenyl)-4-yl]methoxy]-3-oxo-2-(1-piperidinyl)cyclopentyl]-4-heptenoate

To a solution of Intermediate 9 (0.37 g) and dicyclohexylcarbodiimide (0.64 g) in DMSO (8 ml) at 20° was added pyridinium trifluoroacetate (0.254 g) and the mixture stirred for 2 h. Aqueous pH 6 phosphate buffer (75 ml) was added and the mixture extracted with ER (2 × 50 ml). The combined extracts were dried and evaporated and the residue partially purified by chromatography (L) to give an oil (0.224 g). A portion (0.097 g) of this material was converted into its maleate salt by the addition of an excess of an ER solution of maleic acid. The resultant oily precipitate was triturated with ER (5 × 3 ml) and then converted back to the free base by the addition of pH 6 phosphate buffer (10 ml) and extraction with ER (3 × 10 ml). Finally, chromatography (C) afforded pure *title compound* as an oil (0.047 g).

I.R. (Neat) 1740cm$^{-1}$ T.L.C. (L) Rf 0.27.

Example 11

[IR-[1α(Z),2β,5α]]-(−)-(Methylthio)methyl 7-[5-[[(1,1'-Biphenyl)-4-yl]methoxy]-2-(4-morpholinyl)-3-oxocyclopentyl]-4-heptenoate

A solution of Intermediate 10 (0.43 g) in $CH_3CN$ (3 ml) containing triethylamine (0.3 ml) was added to a stirred mixture of chloromethylmethylsulphide (0.15 ml) and NaI (0.05 g) in $CH_3CN$ (1 ml) at room temperature. The mixture was stirred at room temperature for 4h and then kept at 0° overnight. The mixture was diluted with pH 6.5 phosphate buffer (75 ml) and extracted with ER (3 × 50 ml). The combined extracts were dried and evaporated and the residue was purified by chromatography (M) to give the *title compound* as an oil (0.136 g) which solidified on standing, m.p. 48—50°. $[\alpha]_D^{21.5} = -9.0°$ ($CHCl_3$).

I.R. ($CHBr_3$) 1735cm$^{-1}$.

Example 12

[1α(Z),2β,5α]-(±)-3-(Acetylamino)propyl 7-[5-[[(1,1'-Biphenyl)-4-yl]methoxy]-2-(4-morpholinyl)-3-oxocyclopentyl]-4-heptenoate

A solution of Intermediate 1 (0.477 g) in dry $CH_3CN$ (30 ml) containing triethylamine (0.28 ml) was cooled to −10° under dry nitrogen and treated with isobutylchloroformate (0.26 ml). After stirring at −10° for 1h a solution of N-3-hydroxypropylacetamide (0.585 g) in dry pyridine (10 ml) was added and the mixture allowed to warm to room temperature over 2 h. The mixture was poured into pH 6 phosphate buffer (100 ml), extracted into EA (2 × 100 ml), the combined extracts were washed with $H_2O$ (4 × 50 ml), aqueous $CuSO_4$ (2 × 50 ml), $H_2O$ (50 ml) and brine (50 ml), dried and evaporated. Purification of the residue by chromatography (G) gave the *title compound* as an oil (0.103 g).

I.R. (Neat) 3310, 1740, 1680, 1550cm$^{-1}$

| | |
|---|---|
| Analysis Found: | C, 70.4; H, 8.1; N, 4.8; |
| $C_{34}H_{44}N_2O_6$ requires: | C, 70.8; H, 7.7; N, 4.9% |

Example 13

[1R-[1α(Z),2β,5α]]-(−)-(Acetyloxy)methyl 7-[5-[[(1,1'-Biphenyl)-4-yl]methoxy]-2-(4-morpholinyl)-3-oxocyclopentyl]-4-heptenoate

A solution of Intermediate 10 (0.436 g) in acetone (6 ml) containing triethylamine (0.3 ml) and bromomethylacetate (0.3 g) was stirred at 20° for 2h. The mixture was poured into pH 6.5 phosphate buffer (50 ml) and extracted with ER (3 × 50 ml). The combined extracts were dried and evaporated to give an oil (0.45 g). Purification by chromatograhy (C) gave the *title compound* as an oil (0.351 g). $[\alpha]_D^{21.5} = -8.8°$ ($CHCl_3$).

I.R. (Neat) 1765, 1740cm$^{-1}$

| | |
|---|---|
| Analysis Found: | C, 69.8; H, 7.3; N, 2.4; |
| $C_{32}H_{39}NO_7$ requires: | C, 69.9; H, 7.15; N, 2.55% |

Example 14

[1α(Z),2β,5α]-(±)-2-Amino-2-oxoethyl 7-[5-[[(1,1′-Biphenyl)-4-yl]methoxy]-2-(4-morpholinyl)-3-oxocyclopentyl]-4-heptenoate

A mixture of Intermediate 1 (0.3 g), NaI (0.12 g), chloroacetamide (0.294 g) and dicyclohexylamine (0.12 ml) in dry DMF (3 ml) was stirred under nitrogen for 20h. The suspension was poured into pH 6.5 buffer solution (30 ml) and extracted with EA (3 × 25 ml). The combined extracts were washed with brine (30 ml), dried, filtered and evaporated to afford a mobile oil (0.7 g). Chromatography (E) gave impure *title compound* (0.272 g) as an oil. The oil was dissolved EA (20 ml) washed with $H_2O$ (30 ml) and brine (30 ml), dried, filtered and evaporated to give a viscous oil (0.2 g). On taking up into EA/PE (b.p. 60—80°) the *title compound* crystallised out (0.157 g), m.p. 94—95°.

I.R. ($CHBR_3$) 3250, 3400, 1735, 1690, 1665cm$^{-1}$.

Example 15

[1α(Z),2β,5α]-(±)-S-(2-Pyridinyl) 7-[5-[[(1,1′-Biphenyl)-4-yl]methoxy]-2-(4-morpholinyl)-3-oxocyclopentyl]-4-heptenethioate

2,2-Dipyridyldisulphide (0.070 g) was added to a stirred solution of Intermediate 1 (0.1 g) and triphenylphosphine (0.084 g) in benzene (0.6 ml). After 3 days the mixture was poured into pH 6.5 buffer solution (30 ml) and extracted into EA (3 × 25 ml). The combined extracts were dried, filtered and evaporated, and the residue purified by chromatography (C) to give the *title compound* as an oil (0.1 g).

I.R. (Neat) 1738, 1705cm$^{-1}$ T.L.C. (C) Rf 0.24.

Example 16

*a) [1α(Z),2β,5α]-(±)-Methoxymethyl 7-[5-[3-[(1,1′-Biphenyl)-4-yl]propoxy]-2-(4-morpholinyl)-3-oxocyclopentyl]-4-heptenoate*

Pyridine sulphur trioxide complex (0.193 g) in dry DMSO (2 ml) was added to a cold (0°) mixture of Intermediate 22 (0.22 g), triethylamine (0.45 ml), dry DMSO (2 ml) and dry $CH_2Cl_2$ (2 ml). After stirring for 1.5 h $H_2O$ (4 ml) was added and excess $CH_2Cl_2$ removed *in vacuo*. The residue was treated with citric acid (0.22 g) in $H_2O$ (4 ml) and extracted with EA (3 × 20ml). The combined extracts were dried, filtered and evaporated and the residue purified by chromatography (N) to give pure *title compound* as an oil (0.175 g).

I.R. ($CHBr_3$) 1735cm$^{-1}$

| | |
|---|---|
| Analysis Found: | C, 71.8; H, 8.0; N, 2.5; |
| $C_{33}H_{43}NO_6$ requires: | C, 72.1; H, 7.9; N, 2.6% |

The following compounds were similarly prepared:

*b) [1α(Z),2β,5α]-(±)-(Methylthio)methyl 9 - [5 - [[(1,1′ - Biphenyl) - 4 - yl]methoxy] - 2 - (4-morpholinyl) - 3 - oxocyclopentyl] - 6 - nonenoate,* from Intermediate 23 (0.35 g). Purification by chromotography (Y) gave an oil (0.166 g).

I.R. (Neat) 1740cm$^{-1}$

| | |
|---|---|
| Analysis Found: | C, 70.0; H, 7.9; N, 2.4; |
| $C_{33}H_{43}NO_5S$ requires: | C, 70.0; H, 7.7; N, 2.5% |

*c) [1α(Z),2β,5α]-(±) - (Acetylamino)phenyl 7 - [5 - [[3′ - Methoxy(1,1′ - biphenyl) - 4 - yl]methoxy] - 2 - (4-morpholinyl) - 3 - oxocyclopentyl] - 4 - heptenoate,* from Intermediate 24 (0.206 g). Purification by chromatography initially (C) followed by (E) gave a foam (0.182 g).

I.R. ($CHBr_3$) 3420, 1755, 1740, 1690, 1520cm$^{-1}$ T.L.C. (E) Rf 0.26.

*d) [1α(Z),2β,5α]-(±) - 4 - Methylphenyl 7 - [2 - (4-Morpholinyl) - 3 - oxo - 5 - [(2-phenylthien - 4 - yl)methoxy]cyclopentyl] - 4 - heptenoate,* m.p. 78—80° from Intermediate 25 (0.21 g). Purification by chromatography (Z) gave an oil (0.104 g) which solidified on standing.

I.R. (Nujol) 1760, 1742cm$^{-1}$

| | |
|---|---|
| Analysis Found: | C, 70.7; H, 6.9; N, 2.1; |
| $C_{34}H_{39}NO_5S$ requires: | C, 71.2; H, 6.85; N, 2.4% |

*e) [1α(Z),2β,5α]-(±) - 4 - (Methylsulphonyl)phenyl 7 - [5 - [[4′ - Methyl (1,1′ - biphenyl) - 4 - yl]methoxy] - 3 - oxo - 2 - (4 - thiomorpholinyl)cyclopentyl] - 4 - heptenoate, S-dioxide,* from Intermediate 26 (0.312 g). Purification by chromatography (I) gave a foam (0.191 g).

I.R. ($CHBr_3$) 1760, 1745cm$^{-1}$

| | |
|---|---|
| Analysis Found: | C, 63.6; H, 6.2; N, 1.8; |
| $C_{27}H_{43}NO_8S_2$ requires: | C, 64.0; H, 6.2; N, 2.0% |

*f) [1α(Z),2β,5α] - (±) - L - (−) - 1 - (Ethoxycarbonyl)ethyl 7 - [5 - [[(1,1′ - Biphenyl) - 4 - yl]methoxy] - 3 - oxo - 2 - (1 - piperidinyl)cyclopentyl] - 4 - heptenoate,* from the product mixture of Intermediate 27 (0.2 g). Purification by chromatography (AA) gave an oil (0.046 g).

I.R. ($CHBr_3$) 1740(sh), 1732cm$^{-1}$

| | |
|---|---|
| Analysis Found: | C, 73.0; H, 8.1; N, 2.7; |
| $C_{35}H_{45}NO_6$ requires: | C, 73.0; H, 7.9; N, 2.4% |

*g) [1α(Z),2β,5α-(±) - (Acetylthio)methyl 7 - [5 - [[(1,1' - Biphenyl) - 4 - yl]methoxy] - 3 - oxo - 2 - (1 - piperidinyl)cyclopentyl] - 4 - heptenoate,* from Intermediate 28 (0.089 g). Chromatography initially (L) followed by (V) gave an oil (0.05 g).

I.R. ($CHBr_3$) 1733, 1700cm$^{-1}$ T.L.C. (C) Rf 0.59

*h) [1α(Z),2β,5α]-(±) - (Acetyloxy)methyl 7 - [2 - (4 - Morpholinyl) - 3 - oxo - 5 - [4 - (thien - 2 - yl)phenylmethoxy]cyclopentyl] - 4 - heptenoate,* m.p. 78—80.5° from Intermediate 21c. Purification initially by chromatography (BB) and then by recrystallisation from ER/isopentane.

I.R. ($CHBr_3$) 1740cm$^{-1}$

*i) [1α(Z),2β,5α]-(±) - 2 - (4 - Bromophenyl - 2 - oxoethyl 7 - [2 - (4 - Morpholinyl) - 5 - (2 - naphthalenylmethoxy) - 3 - oxocyclopentyl] - 4 - heptenoate,* from Intermediate 29 (0.889 g). Purification by chromatography (CC) gave an oil (0.704 g).

I.R. (Neat) 1745, 1708cm$^{-1}$

Analysis Found: C, 64.8; H, 6.0; N, 1.9;

$C_{35}H_{38}BrNO_6$ requires: C, 64.8; H, 5.9; N, 2.2%

Example 17

[1α(Z),2β,5α]-(±)-(Benzoyloxy)methyl 7-[5-[[(1,1'-Biphenyl)-4-yl]methoxy]-2-(4-morpholinyl)-3-oxocyclopentyl]-4-heptenoate

To a solution of Intermediate 1 (0.477 g), sodium iodide (0.3 g) and triethylamine (0.3 ml) in acetone (5 ml) was added chloromethylbenzoate (0.34 g). After 4h further quantities of chloromethylbenzoate (0.68 g) and triethylamine (0.6 ml) were added and stirring maintained for 20h. The reaction mixture was poured into pH 6.5 phosphate buffer (50 ml) and partitioned with $CH_2Cl_2$ (3 × 50 ml). The dried extracts were evaporated to give a semi-solid (0.95 g). Purification by chromatography (EE) gave the *title compound* as an oil (0.31 g).

I.R. (Neat) 1740cm$^{-1}$

Analysis Found: C, 73.1; H, 7.15; N, 2.1;

$C_{37}H_{41}NO_7$ requires: C, 72.65; H, 6.8; N, 2.3%

Example 18

[1α(Z),2β,5α]-(±)-S-Methoxymethyl 7-[4-[[(1,1'-Biphenyl)-4-yl]methoxy]-2-(4-morpholinyl)-3-oxocyclopentyl]-4-heptenethioate

Isobutylchloroformate (0.19 ml) was added to a solution of Intermediate 1 (0.527 g) and triethylamine (0.4 ml) in dry DMF (4 ml), under nitrogen, at 0°. After 0.5h sodium hydrogen sulphide (0.185 g) was added and the mixture stirred at room temperature for 1h. The suspension was cooled (0°) and triethylamine (0.46 ml) added followed by chloromethyl methyl ether (0.25 ml). After a further 1h the mixture was poured into pH6.5 phosphate buffer (150 ml) and extracted with EA (3 × 50 ml). The dried organic extracts were evaporated *in vacuo* to give an oil (0.72 g). Purification by chromatography (DD) gave the *title compound* as an oil (0.4 g).

I.R. (Neat) 1740, 1695cm$^{-1}$

Analysis Found: C, 68.9; H, 7.4; N, 2.6;

$C_{31}H_{39}NO_5S$ requires: C, 69.2; H, 7.4; N, 2.6%

Example 19

*a) [1α(Z),2β,3β,5α] - (±) - (Methylthio)methyl 7 - [5 - [[(1,1' - Biphenyl) - 4 - yl]methoxy] - 3 - hydroxy - 2 - (4 - morpholinyl)cyclopentyl] - 4 - heptenoate*

Chloromethyl methyl sulphide (0.08 ml) was added to a stirred solution of Intermediate 19 (0.3 g) and dicyclohexylamine (0.14 ml) in dry DMF (3 ml) at room temperature. After 20h the suspension was poured into saturated $NH_4Cl$ solution (30 ml) and extracted with EA (3 × 25 ml). The combined extracts were dried, filtered and evaporated to afford a mobile oil (0.3 g). Column chromatography using (C) as eluent gave the *title compound* as an oil (0.122 g).

TLC (C) $R_f$ 0.26

Analysis Found: C, 68.7; H, 7.7; N, 2.6;

$C_{31}H_{41}NO_5S$ requires: C, 69.0; H, 7.7; N, 2.6%

*b) [1α(Z),2β,3β,5α] and [1α(E),2β,3β,5α] - (±) - (Methylthio)methyl 7 - [3 - Hydroxy - 5 - [[4' - methyl(1,1' - biphenyl) - 4 - yl]methoxy] - 2 - (4 - morpholinyl) cyclopentyl] - 4 - heptenoate,* from Intermediate 67, by the method of Example 19(a). Purification by chromatography (E) gave material of Z:E/2:1

I.R. ($CHBr_3$) 3500—3100, 1730cm$^{-1}$

m/e Found: 554.2955 (m+1)

$C_{32}H_{44}NO_5S$ requires: 554.2940 (m+1)

Example 20

[1α(Z),2β,3β,5α]-(±)-(Acetyloxy)methyl 7-[5-[[(1,1'-Biphenyl)-4-yl]methoxy]-3-hydroxy-2-(4-morpholinyl)-cyclo-pentyl]-4-heptenoate, compound with ethyl acetate (3:1)

To a solution of Intermediate 19 (0.431 g) in pure acetone (4 ml) at 20° was added triethylamine (0.31 ml) followed by bromomethyl acetate (0.305 g) in acetone (2 ml). The mixture was stirred at 20° for 2h and then poured into pH 6 phosphate buffer (75 ml) and extracted with EA (2 × 40 ml). The combined extracts were dried, filtered and concentrated, and the residue purified by chromatography (B) to give the *title compound* as an oil (0.353 g).

TLC (F) $R_f$ 0.3. IR (Neat) 3340 (br), 1760, 1120cm$^{-1}$

Example 21

[1α(Z),2β,3β,5α]-(±)-4-(Acetylamino)phenyl 7-[5-[[(1,1'-Biphenyl)-4-yl]methoxy]-3-hydroxy-2-(4-morpholinyl)cyclopentyl]-4-heptenoate, compound with ethyl acetate (5:1)

To a solution of Intermediate 19 (0.5 g) in pure acetone (10 ml) was added triethylamine (0.58 ml) and the mixture cooled to −10° (ice/EtoH). Isobutylchloroformate (0.41 ml) was added, followed after 0.5h at −10° by 4-(acetylamino)phenol (0.474 g) in acetone (8 ml). After another 0.5h at −10°, pyridine (1 ml) was added and the temperature allowed to rise to 20° over 2h. The mixture was poured into 15% pH 6 phosphate buffer solution (100 ml) and extracted with EA (3 × 50 ml). The combined extracts were dried and evaporated, and the residue purified by chromatography (B) to give the *title compound* as a viscous gum (0.367 g). TLC (FF) $R_f$ 0.43 IR ($CHBr_3$) 3420, 3380, 1750, 1685, 1505 cm$^{-1}$.

Example 22

[1α(Z), 2β,3β,5α]-(±)-(Acetyloxy)methyl 7-[5-[[(1,1'-Biphenyl)-4-yl]methoxy]-3-hydroxy-2-(4-morpholinyl)cyclopentyl]-5-heptenoate.

A solution of the acid derived from Intermediate 31 (0.64 g) in acetone (10 ml) containing triethylamine (0.55 ml) and bromomethyl acetate (0.51 g) was stirred at 20° for 2.5h then poured into pH 6.5 phosphate buffer (150 ml) and extracted with EA (3 × 50 ml). The combined extracts were dried and evaporated and the residue was purified by chromatography (GG) to give the *title compound* as an oil (0.47 g). I.R. ($CHBr_3$) 3500—3100, 1755 cm$^{-1}$.

| | | | |
|---|---|---|---|
| Analysis Found: | C, 69.4; | H, 7.5; | N, 2.5 |
| $C_{32}H_{41}NO_7$ requires: | C, 69.7; | H, 7.5; | N, 2.5%. |

Example 23

a) *[1α(Z),2β,5α] - (±) - (Methylthio)methyl 7 - [5 - [[1,1' - Biphenyl) - 4 - yl]methoxy] - 2 - (4 - morpholinyl) - 3 - oxocyclopentyl] - 5 - heptenoate.*

Pyridinium trifluoroacetate (0.3 g) was added to a stirred solution of Intermediate 33 (0.48 g) and dicyclohexylcarbodiimide (0.82 g) in dry DMSO (4 ml) at ambient temperature. After 5h, the mixture was poured into water (25 ml) and extracted with $CH_2Cl_2$ (3 × 25 ml). The combined extracts were dried and evaporated and the residue was purified by chromatography (H) to give the *title compound* (0.25 g) as a solid, m.p. 45—47°.

| | | | |
|---|---|---|---|
| Analysis Found: | C, 69.7; | H, 7.6; | N, 2.7; |
| $C_{31}H_{39}NO_5S$ requires: | C, 69.3; | H, 7.3; | N, 2.6%. |

The following compounds were prepared by a similar procedure:

b) *[1α(Z),2β,5α] - (±) - (Methylthio)methyl 7 - [5 - [4 - (4 - Methoxybenzoyl)phenylmethoxy] - 2 - (4 - morpholinyl) - 3 - oxocyclopentyl] - 5 - heptenoate,* from Intermediate 37. Purification by chromatography (I). T.L.C. (I) $R_f$ 0.39.

| | | | |
|---|---|---|---|
| Analysis Found: | C, 66.2; | H, 6.95; | N, 2.4; |
| $C_{33}H_{41}NO_7S$ requires: | C, 66.5; | H, 6.90; | N, 2.35%. |

c) *[1α(Z),2β,5α] - (±) - (Methylthio)methyl 7 - [2 - (4 - Morpholinyl) - 3 - oxo - 5 - [4 - (phenylmethyl)phenylmethoxy]cyclopentyl] - 5 - heptenoate,* from Intermediate 40. Purification by chromatography (C) I.R. ($CHBr_3$) 1738 cm$^{-1}$.

| | | | |
|---|---|---|---|
| Analysis Found: | C, 69.6; | H, 7.25; | N, 2.6%; |
| $C_{33}H_{41}NO_5S$ requires: | C, 69.7; | H, 7.5; | N, 2.5%. |

d) *[1α(Z),2β,5α] - (±) - (Methylthio)methyl 7 - [2 - (4 - Morpholinyl) - 3 - oxo - 5 - [[1,1':4',1'' - terphenyl) - 4 - yl]methoxy]cyclopentyl] - 5 - heptenoate,* m.p. 115—118° from Intermediate 41. Purification initially by chromatography (C) and then by crystallisation (EA—PE).

| | | | |
|---|---|---|---|
| Analysis Found: | C, 72.6; | H, 7.0; | N, 2.4; |
| $C_{37}H_{43}NO_5S$ requires: | C, 72.4; | H, 7.1; | N, 2.3%. |

Example 24

a) *[1α(Z),2β,5α] - (±) - 2 - (Dimethylamino)ethyl 7 - [5 - [[(1,1' - Biphenyl) - 4 - yl)methoxy] - 2 - (4 - morpholinyl) - 3 - oxocyclopentyl] - 5 - heptenoate.*

A solution of Intermediate 30 (0.5 g) and triethylamine (0.29 ml) in acetone (10 ml) at −10° was treated with isobutyl chloroformate (0.28 ml) and 15 min later with N,N-dimethyl ethanolamine (0.22 ml). The cooling bath was removed and stirring continued at ambient temperature for 5h. The mixture was poured into 8% $NaHCO_3$ solution (20 ml) and extracted with EA (3 × 30 ml). The combined extracts were washed with brine (50 ml), dried and evaporated. The residue was purified initially by chromatography (O) and then by recrystallisation [ether-PE (b.p. 60—80°)] to give the *title compound* (0.25 g), m.p. 72—73°.

Analysis Found: C, 71.8 H, 7.8; N, 4.8;
$C_{33}H_{44}N_2O_5$ requires: C, 72.2; H, 8.1; N, 5.1%.

The following compounds were prepared by a similar procedure:

b) *[1α(Z),2β,5α] - (±) - Phenyl 7 - [5 - [[(1,1' - Biphenyl) - 4 - yl]methoxy] - 2 - (4 - morpholinyl) - 3 - oxocyclopentyl) - 5 - heptenoate,* m.p. 72.5—74°, from Intermediate 30 and phenol. Purification by chromatography (M).

Analysis Found: C, 75.9; H, 7.1; N, 2.7;
$C_{35}H_{39}NO_5$ requires: C, 75.9; H, 7.1; N, 2.5%.

c) *[1α(Z),2β,5α] - (±) - 4 - Methoxyphenyl 7 - [5 - [[(1,1' - Biphenyl) - 4 - yl]methoxy] - 2 - (4 - morpholinyl) - 3 - oxocyclopentyl] - 5 - heptenoate,* m.p. 75—76.5°, from Intermediate 30 and 4-methoxyphenol. Purification by chromatography (M).

Analysis Found: C, 74.25; H, 7.1; N, 2.4;
$C_{36}H_{41}NO_5$ requires: C, 74.1; H, 7.1; N, 2.4%.

d) *[1α(Z),2β,5α] - (±) - 4 - Acetylphenyl 7 - [5 - [[(1,1' - Biphenyl) - 4 - yl]methoxy] - 2 - (4 - morpholinyl] - 3 - oxocyclopentyl] - 5 - heptenoate,* m.p. 73—75°, from Intermediate 30 and 1-(4-hydroxyphenyl)ethanone. Purification by chromatography (C) and trituration with ER.

Analysis Found: C, 74.75; H, 6.8; N, 2.5;
$C_{37}H_{41}NO_6$ requires: C, 74.6; H, 6.9; N, 2.35%.

e) *[1α(Z),2β,5α] - (±) - 4 - Methylphenyl 7 - [5 - [[(1,1' - Biphenyl) - 4 - yl)]methoxy] - 2 - (4 - morpholinyl) - 3 - oxocyclopentyl] - 5 - heptenoate,* m.p. 81—83°, from Intermediate 30 and 4-methylphenol. Purification by chromatography (M).

Analysis Found: C, 75.9; H, 7.2; N, 2.5;
$C_{36}H_{41}NO_5$ requires: C, 76.15; H, 7.3; N, 2.5%.

f) *[1α(Z),2β,5α] - (±) - 4 - [[7 - [5 - [[(1,1' - Biphenyl) - 4 - yl]methoxy] - 2 - (4 - morpholinyl) - 3 - oxocyclopentyl] - 5 - heptenoyl] - oxy]benzoic acid,* m.p. 95—102° from Intermediate 30 and 4-hydroxybenzoic acid using pyridine as co-solvent. The reaction mixture was pured into 0.1M $KH_2PO_4$ solution and extracted with ER. The ER extract was washed with 0.2M sulphuric acid, pH 6.5 phosphate buffer, dried and evaporated. The residue was purified initially by chromatography (C) and then by crystallisation from ether-isopentane.

Analysis Found: C, 71.9; H, 6.9; N, 2.5;
$C_{36}H_{39}NO_7$ requires: C, 72.3; H, 6.6; N, 2.3%.

Example 25

[1α(Z),2β,5α] - (±) - 2 - Oxo - 2 - phenylethyl 7 - [5 - [[(1,1' - Biphenyl) - 4 - yl]methoxy] - 2 - (4 - morpholinyl) - 3 - oxocyclopentyl] - 5 - heptenoate.

A well stirred solution of Intermediate 38 (0.7 g) in DMSO (5 ml) and triethylamine (5 ml) was treated with pyridine-sulphur trioxide complex (0.65 g) and was kept at ambient temperature for 0.8h. The mixture was poured into saturated $NH_4Cl$ solution (100 ml) and extracted with $CH_2Cl_2$ (3 × 70 ml). The combined extracts were dried and evaporated and the residue was purified by chromatography (C) to give the *title compound* as an oil 0.56 g. I.R. (Neat) 1740, 1700 $cm^{-1}$.

Analysis Found: C, 74.25; H, 6.8; N, 2.3;
$C_{37}H_{41}NO_6$ requires: C, 74.6; H, 6.9; N, 2.35%.

Example 26

[1α(Z),2β,5α)-(±)-4-Acetylaminophenyl 7-[[(1,1'-Biphenyl)-4-yl]methoxy]-2-(4-morpholinyl)-3-oxocyclopentyl]-5-heptenoate, compound with ethyl acetate (10:1).

DMSO (0.61 ml) in $CH_2Cl_2$ (14 ml) was added dropwise to a stirred solution of acetyl bromide (0.57 ml) in $CH_2Cl_2$ (7 ml) under dry nitrogen at −78°. After 10 min. a solution of Intermediate 39 (1.05 g) in $CH_2Cl_2$ (14 ml) was added and stirring continued for 45 min. Triethylamine (2.87 ml) in $CH_2Cl_2$ (14 ml) was added at −78° and the mixture was stirred at ambient temperature for 45 min. The mixture was poured into water (100 ml) and extracted with $CH_2Cl_2$ (3 × 30 ml). The combined extracts were dried and evaporated and the

residue was purified by chromatography (E) to give the *title compound* as a solid (0.73 g), m.p. 45—47°.

Analysis Found: C, 72.9; H, 7.0; N, 4.5;
$C_{37}H_{42}N_2O_6.0.1C_4H_8O_2$ requires: C, 72.5; H, 7.0; N, 4.5%.

Example 27

[1α(Z),2β,5α]-(±)-(Acetyloxy)methyl 7-[5-[[(1,1'-Biphenyl)-4-yl]methoxy]-2-(4-morpholinyl)-3-oxocyclopentyl]-5-heptenoate.

A solution of Intermediate 30 (0.4 g), triethylamine (0.23 ml) and bromomethyl acetate (0.19 g) in dry acetone (6 ml) was stirred at 20° for 2h then poured into pH 6.5 phosphate buffer (20 ml) and extracted with EA (2 × 20 ml). The combined extracts were dried and evaporated, and the residue was purified by chromatography (K) to give the *title compound* as an oil (0.3 g).

I.R. ($CHBr_3$) 1755, 1740 $cm^{-1}$.
Analysis Found: C, 69.7; H, 7.05; N, 2.3;
$C_{32}H_{39}NO_7$ requires: C, 69.9; H, 7.15; N, 2.55%.

Example 28

[1α(Z),2β,5α]-(±)-(3-Pyridinyl) 7-[5-[[(1,1'-Biphenyl)-4-yl]methoxy]-2-(4-morpholinyl)-3-oxocyclopentyl]-5-heptenoate.

A solution of Intermediate 30 (0.35 g) and triethylamine (0.41 ml) in acetone (7.5 ml) at −10° was treated with iso-butylchloroformate (0.29 ml) and 0.25h later with 3-hydroxypyridine (0.35 g). After 0.75h, the solvent was removed *in vacuo* and the residue in pH 6.5 phosphate buffer (30 ml) was extracted with EA (3 x 25 ml). The combined extracts were washed with 0.2N NaOH (30 ml), dried and evaporated, and the residue was purified by chromatography (II) to give the *title compound* as a solid (0.37 g), m.p. 74—75°.

Analysis Found: C, 73.5; H, 6.9; N, 5.0;
$C_{34}H_{38}N_2O_5$ requires: C, 73.6; H, 6.9; N, 5.1%.

Example 29

[1α(Z),2β,5α]-(±)-[4-Aminocarbonyl)phenyl] 7-[5-[[4'-Methoxy(1,1'-biphenyl)-4-yl]methoxy] 2-(4-morpholinyl)-3-oxocyclopentyl]-5-heptenoate.

A well stirred solution of Intermediate 43 (0.395 g) in DMSO (5 ml) and triethylamine (0.7 ml) was treated with pyridine-sulphur trioxide complex (0.3 g) in DMSO (5 ml) and was kept at 20° for 2h. The mixture was poured into pH 6.5 phosphate buffer and extracted with $CH_2Cl_2$. The combined extracts were dried and evaporated, and the residue was purified by chromatography (C through to B) to give the *title compound* to an oil (0.335 g).

I.R. ($CHBr_3$) 3520, 3400, 1753(sh.), 1745, 1680 $cm^{-1}$.
Analysis Found: C, 70.6; H, 7.0; N, 4.8;
$C_{37}H_{42}N_2O_7$ requires: C, 70.9; H, 6.75; N, 4.5%.

Example 30

[1α(Z),2β,5α]-(±)-(Acetylthio)methyl 7-[5-[[(1,1'-Biphenyl)-4-yl]methoxy]-2-(4-morpholinyl-3-oxocyclopentyl]-5-heptenoate.

A well stirred solution of Intermediate 44 (0.31 g) in DMSO (4 ml) and triethylamine (0.6 ml) was treated with pyridine-sulphur trioxide complex (0.26 g) in DMSO (3 ml) and was kept at 20° for 1.5h. The mixture was poured into EA (175 ml) and was washed with pH 6.5 phosphate buffer (3 × 30 ml) and brine (30 ml), then dried and evaporated. The residue was chromatographed twice (T then JJ) to give the pure *title compound* as an oil (0.2 g).

I.R. ($CHBr_3$) 1740, 1700 $cm^{-1}$, T.L.C. (KK) $R_f$ 0.27.

Example 31

[1R-[1α(Z),2β,3β,5α]]-(+)-2-Propenyl 7-[5-[[(1,1'-Biphenyl)-4-yl]methoxy]-3-hydroxy-2-(1-piperidinyl)cyclopentyl]-4-heptenoate.

To a solution of Intermediate 54 (0.4 g) and diisopropylethylamine (0.68 ml) in DMF (5 ml) was added dropwise 3-bromopropene (0.27 ml). The mixture was stirred for 20h then poured into pH 6 phosphate buffer (50 ml) and extracted with EA (3 × 50 ml). The combined extracts were dried and evaporated and the residue purified by chromatography (LL) to give the *title compound* (0.23 g) as an oil.

$[\alpha]_D^{25}$ = +61.9° ($CHCl_3$).
I.R. (Neat) 3400, 1730 $cm^{-1}$.
Analysis Found: C, 76.3; H, 8.7; N, 2.8;
$C_{33}H_{43}NO_4$ requires: C, 76.6; H, 8.4; N, 2.7%.

Example 32

[1R-[1α(Z),2β,3β,5α]]-(+)-Methoxymethyl 7-[5-[[(1,1'-Biphenyl)-4-yl]methoxy]-3-hydroxy-2-(1-piperidinyl)cyclopentyl]-4-heptenoate.

To a cold (0°) solution of Intermediate 54 (0.5 g) and diisopropylethylamine (0.42 ml) in DMF (6 ml) was added dropwise chloromethyl methyl ether (0.11 ml). The mixture was stirred for 3h during which time

ambient temperature was reached. The solution was poured into pH 6 phosphate buffer (50 ml) and extracted with EA (3 × 50 ml). The combined extracts were dried and evaporated and the residue purified by chromatography on alumina [initially (C) then (B)] to give the *title compound* (0.285 g) as an oil.

$[\alpha]_D^{2.5}$ = +60.3° ($CHCl_2$).

I.R. (Neat) 3400, 1745 $cm^{-1}$.

| | | | |
|---|---|---|---|
| Analysis Found: | C, 73.5; | H, 8.5; | N, 2.95; |
| $C_{32}H_{43}NO_5$ requires: | C, 73.7; | H, 8.3; | N, 2.7%. |

Example 33

[1R-[1α(Z), 2β,3β,5α]]-(+)-(2-Oxo-2-phenylethyl) 7-[5-[[(1,1'-Biphenyl)-4-yl]methoxy]-3-hydroxy-2-(1-piperidinyl)cyclopentyl]-4-heptenoate, compound with ethyl acetate (2.5:1).

A solution of Intermediate 54 (0.4 g) in $CH_3CN$ (12 ml) containing diisopropylethylamine (0.4 ml) was treated with α-bromoacetophenone (0.3 g) and stirred for 3h. EA (100 ml) was added and the mixture washed with pH 6.5 phosphate buffer (2 × 40 ml) and brine (40 ml). The organic layer was dried and evaporated, and the residue purified by chromatography (MM) to give the *title compound* (0.4 g) as an oil.

$[\alpha]_D^{23.8}$ = +59.2° ($CHCl_3$).

I.R. (Neat) 1750, 1710 $cm^{-1}$.

| | | | |
|---|---|---|---|
| Analysis Found: | C, 75.4; | H, 7.7; | N, 2.5; |
| $C_{38}H_{45}NO_5.0.4C_4H_8O_2$ requires: | C, 75.4; | H, 7.7; | N, 2.2%. |

Example 34

[1R-[1α(Z),2β,3β,5α]-(+)-(2-Amino-2-oxoethyl) 7-[5-[[(1,1'-Biphenyl)-4-yl]methoxy]-3-hydroxy-2-(1-piperidinyl)cyclopentyl]-4-heptenoate, compound with ethyl acetate (2:1).

A solution of Intermediate 54 (0.5 g) in DMF (7.5 ml) containing diisopropylethylamine (0.5 ml) was treated with chloroacetamide (0.45 g) and sodium iodide (0.25 g) then stirred for 48h. The mixture was poured into pH 6.5 phosphate buffer (80 ml) and extracted with EA (4 × 40 ml). The combined extracts were washed with brine, dried and evaporated, and the residue purified by chromatography [initially (MM) then (RR)] to give the *title compound* as an oil (0.242 g).

$[\alpha]_D^{25}$ = +57.4° ($CHCl_3$).

I.R. ($CHBr_3$) 3520, 3400, 1740, 1695 $cm^{-1}$.

| | | | |
|---|---|---|---|
| Analysis Found: | C, 70.8; | H, 8.4; | N, 5.1; |
| $C_{32}H_{42}N_2O_5.0.5C_4H_8O_2$ requires: | C, 70.6; | H, 8.0; | N, 4.8%. |

Example 35

[1R-[1α(Z),2β,3β,5α]]-(+)-(Methylthio)methyl 7-[5-[[(1,1'-Biphenyl)-4-yl]methoxy]-3-hydroxy-2-(1-piperidinyl)cyclopentyl]-4-heptenoate.

Chloromethylmethyl sulphide (0.15 ml) was added to a stirred mixture of Intermediate 54 (0.5 g), triethylamine (0.75 ml) and sodium iodide (about 5 mg) in dry DMF (7 ml). After 22h further triethylamine (0.75 ml) and chloromethylmethyl sulphide (0.15 ml) were added and stirring continued for 18h. The reaction mixture was diluted with pH 6.5 phosphate buffer (100 ml) and extracted with EA (2 × 50 ml). The combined extracts were washed with brine (40 ml), dried and evaporated, and the residue purified by chromatography [initially (PP) then (QQ)] to give the *title compound* (0.285 g) as an oil.

$[\alpha]_D^{24}$ = +57.8° ($CHCl_3$).

I.R. (Neat) 3400, 1740 $cm^{-1}$.

T.L.C. (LL) $R_f$ 0.28.

Example 36

[1R-[1α(Z),2β,3β,5α]]-(+)-(Acetyloxy)methyl 7-[5-[[(1,1'-Biphenyl)-4-yl]methoxy]-3-hydroxy-2-(1-piperidinyl)cyclopentyl]-4-heptenoate.

Bromomethyl acetate (0.25 ml) was added to an ice cooled, stirred mixture of Intermediate 54 (0.35 g) and triethylamine (0.8 ml) in acetone (8 ml). After 1h the ice bath was removed and the mixture stirred at room temperature for 2.5h. The mixture was diluted with pH 6.5 phosphate buffer (80 ml) and extracted with EA (2 × 50 ml). The organic extracts were washed with brine (50 ml), dried and evaporated, and the residue purified by chromatography (OO) to give the *title compound* (0.224 g) as an oil.

$[\alpha]_D^{26}$ = +59.6° ($CHCl_3$).

I.R. (Neat) 3400, 1760 $cm^{-1}$.

| | | | |
|---|---|---|---|
| Analysis Found: | C, 71.8; | H, 7.9; | N, 2.4; |
| $C_{33}H_{43}NO_6$ requires: | C, 72.1; | H, 7.9; | N, 2.55%. |

Example 37

[1α(Z),2β,3β,5α]-(±)-(Acetyloxy)methyl 7-[3-Hydroxy-5-[[4'-methoxy(1,1'-biphenyl)-4-yl]methoxy]-2-(1-piperidinyl)cyclopentyl]-4-heptenoate.

Bromomethyl acetate (0.06 ml) was added to an ice cooled, stirred solution of Intermediate 62 (0.125 g) and triethylamine (0.1 ml) in acetone (3 ml). After 1h at 0° and 0.5h at room temperature, further triethylamine (0.1 ml) and bromomethyl acetate (0.03 ml) were added. After 1h, the mixture was diluted with pH 6.5 phosphate buffer (30 ml) and extracted with EA (2 × 30 ml). The combined extracts were

washed with brine (20 ml), dried and evaporated, and the residue purified by chromatography [initially (E) then (NN)] to give the *title compound* (0.05 g) as an oil.

I.R. ($CHBr_3$) 1755 $cm^{-1}$.

| | | | |
|---|---|---|---|
| Analysis Found: | C, 70.5; | H, 7.9; | N, 2.6; |
| $C_{34}H_{45}NO_7$ requires: | C, 70.3; | H, 8.0; | N, 2.4%. |

Example 38

[1R-[1α(Z),2β,3β,5α]-(+)-(Acetyloxy)methyl 7-[5-[[(1,1′-Biphenyl)-4-yl]methoxy] 3-hydroxy-2-(1-piperidinyl)cyclopentyl]-5-heptenoate.

To a solution of Intermediate 65 (0.64 g) in acetone (13 ml) was added triethylamine (0.93 ml) followed by a solution of bromomethyl acetate (0.902 g) in acetone (6 ml) and the mixture stirred at 20° for 4h, then stored at 0—5° for 13.5h. The solution was then poured into pH 6 phosphate buffer solution (100 ml) and extracted with $CH_2Cl_2$ (3 × 80 ml). The combined extracts were dried, evaporated *in vacuo,* and the residue purified by column chromatography (PP) to give the *title compound* as an oil (0.313 g).

I.R. ($CHBr_3$) 3500—2600, 2810, 2760, 1760 $cm^{-1}$.

T.L.C. (FF) $R_f$ 0.48.

$[\alpha]_D^{25} = +33.6°$ ($CHCl_3$).

Example 39

[1R-[1α(Z),2β,3β,5α]]-[4-(Acetylamino] 7-[5-[[(1,1′-Biphenyl)-4-yl]methoxy]-3-hydroxy-2-(1-piperidinyl)-cyclopentyl]-4-heptenoate.

Isobutyl chloroformate (0.24 ml) was added under nitrogen to a stirred, cooled (bath temp. −10°) mixture of the Intermediate 54 (0.3 g) and triethylamine (0.47 ml) in pure acetone (7.5 ml). After 20 min, p-acetamidophenol (0.5 g) was added and the mixture stirred at −10° for 2.5h and then at room temperature for 1h.

The mixture was diluted with pH 6.5 phosphate buffer (80 ml) and extracted with EA (2 × 60 ml). The combined organic extracts were washed with brine (30 ml), dried and evaporated *in vacuo* to give a gum (0.76 g). The gum was redissolved in $CH_2Cl_2$ (20 ml), the solid filtered off and the filtrate evaporated *in vacuo.* The residue was purified by chromatography [initially (LL) then (VV)] to give the *title compound* as a foam (0.175 g).

I.R. ($CHBr_3$) 3425, 1750, 1690 $cm^{-1}$.

T.L.C. (WW, double elution) $R_f$ 0.34.

Pharmaceutical Examples

Tablets

These may be prepared by direct compression or wet granulation. The direct compression method is preferred but may not be suitable in all cases as it is dependent upon the dose level and physical characteristics of the active ingredient.

| A. | Direct Compression | mg/tablet |
|---|---|---|
| | Active ingredient | 100.00 |
| | Microcrystalline Cellulose B.P.C. | 298.00 |
| | Magnesium Stearate | 2.00 |
| | Compression Weight | 400.00 mg |

The active ingredient is sieved through a 250 $m^{-6}$ sieve, blended with the excipients and compressed using 10.0 mm punches. Tablets of other strengths may be prepared by altering the compression weight and using punches to suit.

| B. | Wet Granulation | mg/tablet |
|---|---|---|
| | Active ingredient | 100.00 |
| | Lactose B.P. | 238.00 |
| | Starch B.P. | 40.00 |
| | Pregelatinised Maize Starch B.P. | 20.00 |
| | Magnesium Stearate B.P. | 2.00 |
| | Compressed Weight | 400.00 mg |

The active ingredient is sieved through a 250 $m^{-6}$ sieve and blended with the lactose, starch and pregelatinised starch. The mixed powders are moistened with purified water, granules are made, dried, screened and blended with the magnesium stearate. The lubricated granules are compressed into tablets as described for the direct compression formulae. The tablets may be film coated with suitable film forming materials, e.g. methyl cellulose or hydroxylpropyl methyl cellulose using standard techniques. Alternatively the tablets may be sugar coated.

| Capsules | mg/capsule |
|---|---|
| Active ingredient | 100.00 |
| *STA—RX 1500 | 99.00 |
| Magnesium Stearate B.P. | 1.00 |
| Fill Weight | 200.00 mg |

*A form of directly compressible starch supplied by Colorcorn Ltd., Orpington, Kent.

The active ingredient is sieved through a 250 $m^{-6}$ sieve and blended with the other materials. The mix is filled into No. 2 hard gelatin capsules using a suitable filling machine. Other doses may be prepared by altering the fill weight and if necessary changing the capsule size to suit.

| Inhalation Cartridges | /cartridge |
|---|---|
| Active ingredient (micronised) | 3 mg |
| Lactose B.P. to | 25 mg |

The active ingredient is micronised so that the majority of the particles are between 1 $m^{-6}$ and 5 $m^{-6}$ in longest dimensions and none are greater than 10 $m^{-6}$. The active ingredient is then blended with the lactose and the mix is filled into No. 3 hard gelatin capsules using a suitable filling machine.

| Suspensions | mg/5 ml dose |
|---|---|
| Active ingredient | 100.0 |
| Aluminium monostearate | 75.0 |
| Sucrose (powdered) | 125.0 |
| Flavour } Colour } | as required |
| Fractionated coconut oil to | 5.00 ml. |

The aluminium monostearate is dispersed in about 90% of the fractionated coconut oil. The resulting suspension is heated to 115°C while stirring and then cooled. The flavour and colour are added and the active ingredient and sucrose are suitably dispersed. The suspension is made up to volume with the remaining fractionated coconut oil and mixed.

| Injection for Intravenous Administration | |
|---|---|
| Active ingredient | 50 mg |
| Suitable vehicle to | 5 ml. |

A sterile presentation of the active ingredient in an ampoule or vial together with an ampoule containing a suitable vehicle. The former may be prepared by (a) filling sterile material into vials under aseptic conditions (b) freeze drying a sterile solution of the active ingredient under aseptic conditions.

The vehicle may be (a) Water for Injections B.P. (b) Water for Injections B.P. containing: (1) sodium chloride to adjust the tonicity of the solution and/or (2) buffer salts or dilute acid or alkali to facilitate solution of the active ingredient.

The vehicle is prepared, clarified and filled into appropriate sized ampoules sealed by fusion of the glass. The vehicle is sterilised by heating in an autoclave using one of the acceptable cycles.

**Claims for the Contracting States: BE CH DE FR GB IT LI LU NL SE**

1. Compounds of the general formulae (1a) and (1b)

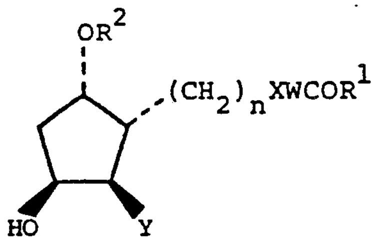

(1a)

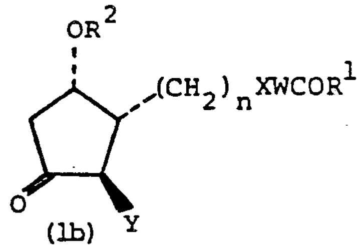

(1b)

wherein $R^1$ is

(a) $-AR^3$ where A is —O— or —S— and $R^3$ is phenyl [optionally substituted by $C_{1-4}$ alkyl, $C_{1-4}$ alkoxy, $C_{1-4}$ alkanoyl, methylthio, methylsulphinyl, methylsulphonyl, halogen, $-CO_2R^4$ [where $R^4$ is a hydrogen atom, $C_{1-4}$ alkyl or phenyl], $-NHCOR^4$, $-CONR^5R^6$ [where $R^5$ and $R^6$ may be the same or different and are each a hydrogen atom or $C_{1-4}$ alkyl], $C_{1-4}$ alkylsulphonylamino, formyl, nitro, cyano, phenyl or $-NR^5R^6$];

(b) $-OCH_2COR^7$ where $R^7$ is phenyl (optionally substituted by a halogen atom, $C_{1-4}$ alkyl or $C_{1-4}$ alkoxy) or $-NH_2$;

(c) $-A(CH_2)_mBR^5$ where A is —O— or —S—, m is 1—3 and B is —O— or —S8, provided that when m is 1, $R^5$ is not a hydrogen atom;

(d) $-A(CH_2)_pR^8$ where A is —O— or —S—, p is 2 or 3 and $R^8$ is an N-attached $C_{1-4}$ dialkylamino, morpholino, piperidino, pyrrolidino, acetylamino or benzoylamino group;

(e) $-OCH(CH_2N(CH_3)_2)_2$

(f)

$$\begin{array}{c} R^9 \\ | \\ -ACHCO_2R^5 \end{array}$$

where A is —O— or —S— and $R^9$ is a hydrogen atom, methyl or phenyl;

(g) $-OCH_2OCOR^{10}$ where $R^{10}$ is $C_{1-4}$ alkyl, methoxy or phenyl;

(h) $-OCH_2SCOR^{11}$ where $R^{11}$ is $C_{1-4}$ alkyl;

(i) pyridinyloxy or pyridinylthio;

(j) 1-(acetyloxy)ethoxy, (acetyloxy)phenylmethoxy, tetrahydro-5-oxo-2-furanyloxy, tetrahydro-2-oxo-3-furanyloxy, triphenylmethoxy or diphenylmethoxy; or

(k) $-OR^{12}$ where $R^{12}$ is $C_{3-6}$ alkenyl, $C_{5-7}$ cycloalkyl (optionally substituted by one or more $C_{1-4}$ alkyl groups), $-CH_2CCl_3$ or furanylmethyl;

n is 1 or 2;

W is straight or branched $C_{1-7}$ alkylene;

X is cis or trans —CH=CH— or $-CH_2CH_2-$;

Y is a saturated heterocyclic amino group (attached to the cyclopentane ring via the nitrogen atom) which has 5—8 ring members and (a) optionally contains in the ring —O—, —S—, $-SO_2-$, or $-NR^{13}-$ (where $R^{13}$ is a hydrogen atom, $C_{1-7}$ alkyl or aralkyl having a $C_{1-4}$ alkyl portion); and/or (b) is optionally substituted by one or more $C_{1-4}$ alkyl groups;

$R^2$ is (i) straight or branched $C_{1-5}$ alkyl substituted by (a) phenyl [optionally substituted by $C_{1-6}$ alkyl, $C_{5-7}$ cycloalkyl, phenylalkyl having a $C_{1-3}$ alkyl portion, thienyl, phenyl (optionally substituted by $C_{1-4}$ alkyl, $C_{1-4}$ alkoxy or phenyl), benzoyl (optionally substituted by $C_{1-4}$ alkyl, $C_{1-4}$ alkoxy, halogen, trifluoromethyl or phenyl) or $C_{5-7}$ cycloalkanoyl], (b) thienyl [optionally substituted by $C_{5-7}$ cycloalkyl or phenyl (optionally substituted by $C_{1-3}$ alkyl, $C_{1-3}$ alkoxy or halogen)], or (c) naphthyl (optionally substituted by $C_{1-4}$ alkyl or $C_{1-4}$ alkoxy), or (ii) cinnamyl (optionally substituted by benzoyl); and the physiologically acceptable salts and solvates thereof.

2. Compounds as claimed in claim 1 in which Y is morpholino or piperidino.

3. Compounds as claimed in any one of the preceding claims in which X is cis —CH=CH—.

4. Compounds as claimed in any one of the preceding claims in which n is 2 and W is $-CH_2CH_2-$ or $-CH_2CH_2CH_2CH_2-$.

5. Compounds as claimed in any one of the preceding claims in which $R^2$ is a benzyl group in which the phenyl group is substituted by thienyl or phenyl, which latter phenyl is optionally substituted by $C_{1-4}$ alkyl or $C_{1-4}$ alkoxy.

6. Compounds as claimed in any one of the preceding claims in which $R^1$ is $-OCH_2OCOCH_3$, $-OCH_2SCH_3$, $-O(CH_2)_3NHCOCH_3$, $-OCH_2CONH_2$, 4-acetamidophenoxy or allyloxy.

7. Compounds as claimed in claim 1 in which:
$R^1$ is $—OCH_2OCOCH_3$, $—OCH_2SCH_3$, $—O(CH_2)_3NHCOCH_3$, $—OCH_2CONH_2$, 4-acetamidophenoxy or allyloxy
W is $—CH_2CH_2—$,
n is 2,
X is cis —CH=CH—
Y is morpholino or piperidino in compounds of formula (1b) or in piperidino in compounds of formula (1a), and
$R^2$ is benzyl in which the phenyl group is substituted by phenyl, tolyl or methoxyphenyl, and the physiologically acceptable salts and solvates thereof.

8. Compounds as claimed in any one of the preceding claims in which the carbon atom carrying the $—(CH_2)_nXWCOR^1$ group is in the R— configuration.

9. Compounds as claimed in any one of the preceding claims, said compounds having the formula (1a).

10. A pharmaceutical composition comprising a compound as claimed in any one of the preceding claims and one or more pharmaceutical carriers.

11. A process for the preparation of a compound as claimed in claim 1 which comprises:
(a) esterifying the corresponding compound in which $R^1$ is —OH, or, in the preparation of a compound in which $R^1$ is a group of the type (c) and A is —S—, reacting the corresponding compound in which $R^1$ is —SH with a halide, $R^5BCH_2Hal$;
(b) reducing the corresponding compound in which X is an acetylene group;
(c) reducing the corresponding compound in which $R^3$ is phenyl substituted azido to prepare a compound in which $R^3$ is phenyl substituted by amino;
(d) in the preparation of a compound in which X is $—CH_2CH_2—$, catalytically hydrogenating a corresponding compound in which X is —CH=CH—;
(e) in the preparation of a compound of formula (1b), oxidising a corresponding hydroxy compound of formula (4)

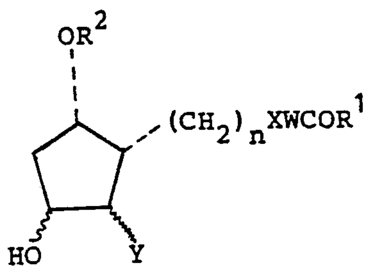

(4)

(f) in the preparation of a compound of formula (1b) in which $R^1$ is $—OCH_2SCH_3$, simultaneously oxidising and esterifying the corresponding compound of formula (4) in which $R^1$ is —OH; or
(g) in the preparation of a salt, treating a compound of formula (1a) or (1b) with an acid, or, when $R^1$ contains a —COOH group, with a base.

**Claims for the Contracting State: AT**

1. A process for the preparation of a compound of the general formula (1a) or (1b)

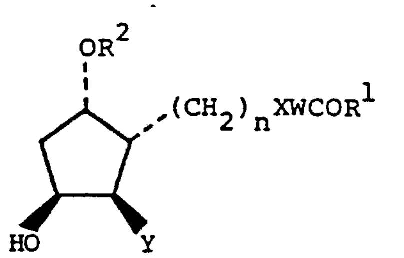

(1a)

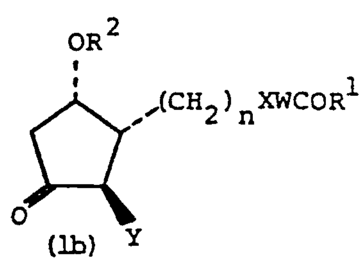

(1b)

wherein $R^1$ is
(a) $—AR^3$ where A is —O— or —S— and $R^3$ is phenyl [optionally substituted by $C_{1-4}$ alkyl, $C_{1-4}$ alkoxy, $C_{1-4}$ alkanoyl, methylthio, methylsulphinyl, methylsulphonyl, halogen, $—CO_2R^4$ [where $R^4$ is a hydrogen atom, $C_{1-4}$ alkyl or phenyl], $—NHCOR^4$, $—CONR^5R^6$ [where $R^5$ and $R^6$ may be the same or different and are each a hydrogen atom or $C_{1-4}$ alkyl], $C_{1-4}$ alkylsulphonylamino, formyl, nitro, cyano, phenyl or $—NR^5R^6$];

(b) $—OCH_2COR^7$ where $R^7$ is phenyl (optionally substituted by a halogen atom, $C_{1-4}$ alkyl or $C_{1-4}$ alkoxy) or $—NH_2$;

(c) $—A(CH_2)_mBR^5$ where A is —O— or —S—, m is 1—3 and B is —O— or —S—, provided that when m is 1, $R^5$ is not a hydrogen atom;

(d) $—A(CH_2)_pR^8$ where A is —O— or —S—, p is 2 or 3 and $R^8$ is an N-attached $C_{1-4}$ dialkylamino, morpholino, piperidino, pyrrolidino, acetylamino or benzoylamino group;

(e) $—OCH(CH_2N(CH_3)_2)_2$

(f)

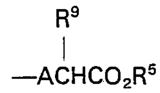

where A is —O— or —S— and $R^9$ is a hydrogen atom, methyl or phenyl;

(g) $—OCH_2OCOR^{10}$ where $R^{10}$ is $C_{1-4}$ alkyl, methoxy or phenyl;

(h) $—OCH_2SCOR^{11}$ where $R^{11}$ is $C_{1-4}$ alkyl;

(i) pyridinyloxy or pyridinylthio;

(j) 1-(acetyloxy)ethoxy, (acetyloxy)phenylmethoxy, tetrahydro-5-oxo-2-furanyloxy, tetrahydro-2-oxo-3-furanyloxy, triphenylmethoxy or diphenylmethoxy; or

(k) $—OR^{12}$ where $R^{12}$ is $C_{3-6}$ alkenyl, $C_{5-7}$ cycloalkyl (optionally substituted by one or more $C_{1-4}$ alkyl groups), $—CH_2CCl_3$ or furanylmethyl;

n is 1 or 2;

W is straight or branched $C_{1-7}$ alkylene;

X is cis or trans —CH=CH— or $—CH_2CH_2—$;

Y is a saturated heterocyclic amino group (attached to the cyclopentane ring via the nitrogen atom) which has 5—8 ring members and (a) optionally contains in the ring —O—, —S—, $—SO_2—$, or $—NR^{13}—$ (where $R^{13}$ is a hydrogen atom, $C_{1-7}$ alkyl or aralkyl having a $C_{1-4}$ alkyl portion); and/or (b) is optionally substituted by one or more $C_{1-4}$ alkyl groups;

$R^2$ is (i) straight or branched $C_{1-5}$ alkyl substituted by (a) phenyl [optionally substituted by $C_{1-6}$ alkyl, $C_{5-7}$ cycloalkyl, phenylalkyl having a $C_{1-3}$ alkyl portion, thienyl, phenyl (optionally substituted by $C_{1-4}$ alkyl, $C_{1-4}$ alkoxy or phenyl), benzoyl (optionally substituted by $C_{1-4}$ alkyl, $C_{1-4}$ alkoxy, halogen, trifluoromethyl or phenyl) or $C_{5-7}$ cycloalkanoyl], (b) thienyl [optionally substituted by $C_{5-7}$ cycloalkyl or phenyl (optionally substituted by $C_{1-3}$ alkyl, $C_{1-3}$ alkoxy or halogen)], or (c) naphthyl (optionally substituted by $C_{1-4}$ alkyl or $C_{1-4}$ alkoxy), or (ii) cinnamyl (optionally substituted by benzoyl); and the physiologically acceptable salts and solvates thereof, which process comprises:

(a) esterifying the corresponding compound in which $R^1$ is —OH, or, in the preparation of a compound in which $R^1$ is a group of the type (c) and A is —S—, reacting the corresponding compound in which $R^1$ is —SH with a halide, $R^5BCH_2Hal$;

(b) reducing the corresponding compound in which X is an acetylene group;

(c) reducing the corresponding compound in which $R^3$ is phenyl substituted by azido to prepare a compound in which $R^3$ is phenyl substituted by amino;

(d) in the preparation of a compound in which X is $—CH_2CH_2—$, catalytically hydrogenating a corresponding compound in which X is —CH=CH—;

(e) in the preparation of a compound of formula (1b), oxidising a corresponding hydroxy compound of formula (4)

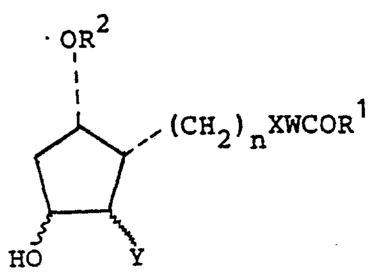

(4)

(f) in the preparation of a compound of formula (1b) in which $R^1$ is $—OCH_2SCH_3$, simultaneously oxidising and esterifying the corresponding compound of formula (4) in which $R^1$ is —OH; or

(g) in the preparation of a salt, treating a compound of formula (1a) or (1b) with an acid, or, when $R^1$ contains a —COOH group, with a base.

2. A process as claimed in claim 1 in which Y in the product is morpholino or piperidino.

3. A process as claimed in any one of the preceding claims in which X in the product in cis —CH=CH—.

4. A process as claimed in any one of the preceding claims in which, in the product, n is 2 and W is $-CH_2CH_2-$ or $-CH_2CH_2CH_2CH_2-$.

5. A process as claimed in any one of the preceding claims in which $R^2$ in the product is a benzyl group in which the phenyl group is substituted by thienyl or phenyl, which latter phenyl is optionally substituted by $C_{1-4}$ alkyl or $C_{1-4}$ alkoxy.

6. A process as claimed in any one of the preceding claims in which $R^1$ in the product is $-OCH_2OCOCH_3$, $-OCH_2SCH_3$, $-O(CH_2)_3NHCOCH_3$, $-OCH_2CONH_2$, 4-acetamidophenoxy or allyloxy.

7. A process as claimed in claim 1 in which the product is a compound of formula (1a) or (1b) in which:

$R^1$ is $-OCH_2OCOCH_3$, $-OCH_2SCH_3$, $-O(CH_2)_3NHCOCH_3$, $-OCH_2CONH_2$, 4-acetamidophenoxy or allyloxy

W is $-CH_2CH_2-$,

n is 2,

X is cis $-CH{=}CH-$

Y is morpholino or piperidino in compounds of formula (1b) or in piperidino in compounds of formula (1a), and

$R^2$ is benzyl in which the phenyl group is substituted by phenyl, tolyl or methoxyphenyl, or a physiologically acceptable salt or solvate thereof.

8. A process as claimed in any one of the preceding claims in which the carbon atom carrying the $-(CH_2)_nXWCOR^1$ group is in the R— configuration in the product.

9. A process as claimed in any one of the preceding claims in which the product is a compound of formula (1a).

10. A process for the manufacture of a pharmaceutical composition comprising a compound of formula (1) as defined in any of the preceding claims, which comprises formulating said compound with one or more pharmaceutical carriers.

11. A process as claimed in claim 10 in which a unit dose of the compound of formla (1) is formulated as a tablet or liquid for injection, or in which the compound of formula (1) is formulated as an aerosol, solution or inhalation cartridge for the inhalation of a metered amount of said compound.

**Patentansprüche für die Vertragsstaaten: BE CH DE FR GB IT LI LU NL SE**

1. Verbindungen der allgemeinen Formeln (1a) und (1b)

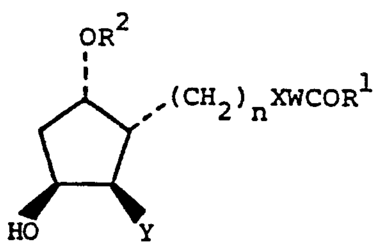

(1a)

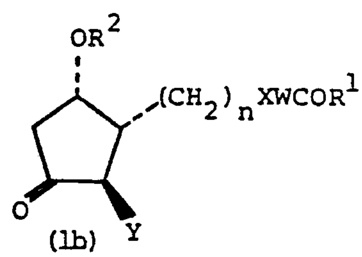

(1b)

worin $R^1$ ist

(a) $-AR^3$, worin A ist —O— oder —S— und $R^3$ Phenyl ist [gegebenenfalls substituiert durch $C_{1-4}$-Alkyl, $C_{1-4}$-Alkoxy, $C_{1-4}$-Alkanoyl, Methylthio, Methylsulfinyl, Methylsulfonyl, Halogen, $-CO_2R^4$ (worin $R^4$ ein Wasserstoffatom, $C_{1-4}$-Alkyl oder Phenyl ist), $-NHCOR^4$, $-CONR^5R^6$ (worin $R^5$ und $R^6$ gleich oder verschieden sein können und jeweils ein Wasserstoffatom oder $C_{1-4}$-Alkyl sind), $C_{1-4}$-Alkylsulfonylamino, Formyl, Nitro, Cyano, Phenyl oder $-NR^5R^6$];

(b) $-OCH_2COR^7$, worin $R^7$ Phenyl ist (gegebenenfalls substituiert durch ein Halogenatom, $C_{1-4}$-Alkyl oder $C_{1-4}$-Alkoxy) oder $-NH_2$;

(c) $-A(CH_2)_mBR^5$, worin A ist —O— oder —S—, m ist 1—3 und B ist —O— oder —S—, vorausgesetzt, daß, falls m = 1, $R^5$ nicht ein Wasserstoffatom ist;

(d) $-A(CH_2)_pR^8$, worin A ist —O— oder —S—, P ist 2 oder 3 und $R^8$ ist eine N-verknüpfte $C_{1-4}$-Dialkylamino-, Morpholino-, Piperidino-, Pyrrolidino-, Acetylamino- oder Benzoylaminogruppe;

(e) $-OCH(CH_2N(CH_3)_2)_2$

(f)

$$\begin{array}{c} R^9 \\ | \\ -ACHCO_2R^5, \end{array}$$

worin A ist —O— oder —S— und $R^9$ ein Wasserstoff atom, Methyl oder Phenyl ist;

(g) $-OCH_2OCOR^{10}$, worin $R^{10}$ $C_{1-4}$-Alkyl, Methoxy oder Phenyl ist;

(h) $-OCH_2SCOR^{11}$, worin $R^{11}$ $C_{1-4}$-Alkyl ist;

(i) Pyridinyloxy oder Pyridinylthio;

(j) 1-(Acetyloxy)-ethoxy, (Acetyloxy)-phenylmethoxy, Tetrahydro-5-oxo-2-furanyloxy, Tetrahydro-2-oxo-3-furanyloxy, Triphenylmethoxy oder Diphenylmethoxy; oder

(k) $-OR^{12}$, worin $R^{12}$ ist $C_{3-6}$-Alkenyl, $C_{5-7}$-Cycloalkyl (gegebenenfalls substituiert durch eine oder mehrere $C_{1-4}$-Alkylgruppen), $-CH_2CCl_3$ oder Furanylmethyl;

n ist 1 oder 2;

W ist geradkettiges oder verzweigtes $C_{1-7}$-Alkylen;

X ist cis- oder trans-$CH=CH-$ oder $-CH_2CH_2-$;

Y ist eine gesättigte heterocyclische Aminogruppe (verknüpft mit dem Cyclopentanring über das Stickstoffatom), welche 5 bis 8 Ringglieder hat und (a) gegebenenfalls im Ring $-O-$, $-S-$, $-SO_2-$ oder $-NR^{13}-$ enthält (worin $R^{13}$ ein Wasserstoffatom, $C_{1-7}$-Alkyl oder Aralkyl mit einem $C_{1-4}$-Alkylteil ist); und/oder (b) gegebenenfalls substituiert ist durch eine oder mehrere $C_{1-4}$-Alkylgruppen;

$R^2$ ist (i) geradkettiges oder verzweigtes $C_{1-5}$-Alkyl substituiert durch (a) Phenyl [gegebenenfalls substituiert durch $C_{1-6}$-Alkyl, $C_{5-7}$-Cycloalkyl, Phenylalkyl mit einem $C_{1-3}$-Alkylteil, Thienyl, Phenyl (gegebenenfalls substituiert durch $C_{1-4}$-Alkyl, $C_{1-4}$-Alkoxy oder Phenyl), Benzoyl (gegebenenfalls substituiert durch $C_{1-4}$-Alkyl, $C_{1-4}$-Alkoxy, Halogen, Trifluormethyl oder Phenyl) oder $C_{5-7}$-Cycloalkanoyl], (b) Thienyl [gegebenenfalls substituiert durch $C_{5-7}$-Cycloalkyl oder Phenyl (gegebenenfalls substituiert durch $C_{1-3}$-Alkyl, $C_{1-3}$-Alkoxy oder Halogen)], oder (c) Naphthyl (gegebenenfalls substituiert durch $C_{1-4}$-Alkyl oder $C_{1-4}$-Alkoxy), oder (ii) Cinnamyl (gegebenenfalls substituiert durch Benzoyl); und die physiologisch annehmbaren Salze und Derivate davon.

2. Verbindungen gemäß Anspruch 1, worin Y Morpholino oder Piperidino ist.

3. Verbindungen gemäß einem der vorhergehenden Ansprüche, worin X cis-$CH=CH-$ ist.

4. Verbindungen gemäß einem der vorhergehenden Ansprüche, worin n = 2 und W ist $-CH_2CH_2-$ oder $-CH_2CH_2CH_2CH_2-$.

5. Verbindungen gemäß einem der vorhergehenden Ansprüche, worin $R^2$ eine Benzylgruppe ist, worin die Phenylgruppe substituiert ist durch Thienyl oder Phenyl, welch letzteres Phenyl gegebenenfalls substituiert ist durch $C_{1-4}$-Alkyl oder $C_{1-4}$-Alkoxy.

6. Verbindungen gemäß einem der vorhergehenden Ansprüche, worin $R^1$ ist $-OCH_2OCOCH_3$, $-OCH_2SCH_3$, $-O(CH_2)_3NHCOCH_3$, $-OCH_2CONH_2$, 4-Acetamidophenoxy oder Allyloxy.

7. Verbindungen gemäß Anspruch 1, worin:

$R^1$ ist $-OCH_2OCOCH_3$, $-OCH_2SCH_3$, $-O(CH_2)_3NHCOCH_3$, $-OCH_2CONH_2$, 4-Acetamidophenoxy oder Allyloxy,

$W = -CH_2CH_2-$,

$n = 2$,

X ist cis-$CH=CH-$,

Y ist Morpholino oder Piperidino in Verbindungen der Formel (1b) oder ist Piperidino in Verbindungen der Formel (1a), und

$R^2$ ist Benzyl, worin die Phenylgruppe durch Phenyl, Tolyl oder Methoxyphenyl substituiert ist, und die physiologisch annehmbaren Salze und Solvate davon.

8. Verbindungen gemäß einem der vorhergehenden Ansprüche, worin das die Gruppe $-(CH_2)_nXWCOR^1$ tragende Kohlenstoffatom in der R-Konfiguration vorliegt.

9. Verbindungen gemäß einem der vorhergehenden Ansprüche, wobei diese Verbindungen die Formel (1a) haben.

10. Eine pharmazeutische Zusammensetzung, umfassend eine Verbindung, wie in einem der vorhergehenden Ansprüche beansprucht und einen oder mehrere pharmazeutische Träger.

11. Verfahren zur Herstellung einer Verbindung gemäß Anspruch 1, dadurch gekennzeichnet:

(a) daß man die entsprechende Verbindung, worin $R^1$ $-OH$ ist, verestert oder, zur Herstellung einer Verbindung, worin $R^1$ eine Gruppe vom Typ (c) ist und A $-S-$ ist, die entsprechende Verbindung, worin $R^1$ $-SH$ ist, mit einem Halogenid, $R^5BCH_2Hal$, umsetzt;

(b) daß man die entsprechende Verbindung, worin X eine Acetylengruppe ist, reduziert;

(c) daß man die entsprechende Verbindung, worin $R^3$ Phenyl, substituiert durch Azido, ist, zur Herstellung einer Verbindung, worin $R^3$ Phenyl, substituiert durch Amino, ist, reduziert;

(d) daß man zur Herstellung einer Verbindung, worin X $-CH_2CH_2-$ ist, eine entsprechende Verbindung, worin X $-CH=CH-$ ist, katalytisch hydriert;

(e) daß man zur Herstellung einer Verbindung der Formel (1b) eine entsprechende Hydroxyverbindung der Formel (4)

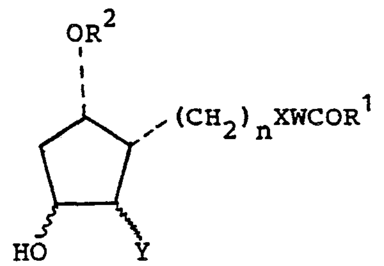

(4)

oxidiert.

(f) daß man zur Herstellung einer Verbindung der Formel (1b), worin $R^1$ —$OCH_2SCH_3$ ist, die entsprechende Verbindung der Formel (4), worin $R^1$ —OH ist, gleichzeitig oxidiert und verestert; oder

(g) daß man zur Herstellung eines Salzes eine Verbindung der Formel (1a) oder (1b) mit einer Säure, oder, wenn $R^1$ eine —COOH-Gruppe enthält, mit einer Base behandelt.

**Patentansprüche für den Vertragsstaat: AT**

1. Verfahren zur Herstellung einer Verbindung der allgemeinen Formel (1a) oder (1b)

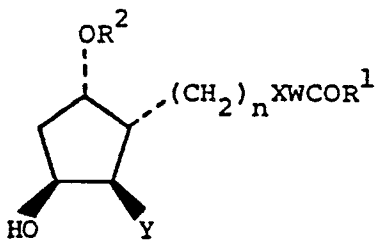

(1a)

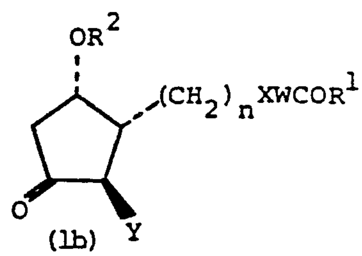

(1b)

worin $R^1$ ist

(a) —$AR^3$, worin A ist —O— oder —S— und $R^3$ Phenyl ist [gegebenenfalls substituiert durch $C_{1-4}$-Alkyl, $C_{1-4}$-Alkoxy, $C_{1-4}$-Alkanoyl, Methylthio, Methylsulfinyl, Methylsulfonyl, Halogen, —$CO_2R^4$ (worin $R^4$ ein Wasserstoffatom, $C_{1-4}$-Alkyl oder Phenyl ist), —$NHCOR^4$, —$CONR^5R^6$ (worin $R^5$ und $R^6$ gleich oder verschieden sein können und jeweils ein Wasserstoffatom oder $C_{1-4}$-Alkyl sind), $C_{1-4}$-Alkylsulfonylamino, Formyl, Nitro, Cyano, Phenyl oder —$NR^5R^6$];

(b) —$OCH_2COR^7$, worin $R^7$ Phenyl ist (gegebenenfalls substituiert durch ein Halogenatom, $C_{1-4}$-Alkyl oder $C_{1-4}$-Alkoxy) oder —$NH_2$;

(c) —$A(CH_2)_mBR^5$, worin A ist —O— oder —S—, m ist 1—3 und B ist —O— oder —S—, vorausgesetzt, daß, falls m = 1, $R^5$ nicht ein Wasserstoffatom ist;

(d) —$A(CH_2)_pR^8$, worin A ist —O— oder —S—, P ist 2 oder 3 und $R^8$ ist eine N-verknüpfte $C_{1-4}$-Dialkylamino-, Morpholino-, Piperidino-, Pyrrolidino-, Acetylamino- oder Benzoylaminogruppe;

(e) —$OCH(CH_2N(CH_3)_2)_2$

(f)

$$\begin{array}{c} R^9 \\ | \\ -ACHCO_2R^5, \end{array}$$

worin A ist —O— oder —S— und $R^9$ ein Wasserstoff atom, Methyl oder Phenyl ist;

(g) —$OCH_2OCOR^{10}$, worin $R^{10}$ $C_{1-4}$-Alkyl, Methoxy oder Phenyl ist;

(h) —$OCH_2SCOR^{11}$, worin $R^{11}$ $C_{1-4}$-Alkyl ist;

(i) Pyridinyloxy oder Pyridinylthio;

(j) 1-(Acetyloxy)-ethoxy, (Acetyloxy)-phenylmethoxy, Tetrahydro-5-oxo-2-furanyloxy, Tetrahydro-2-oxo-3-furanyloxy, Triphenylmethoxy oder Diphenylmethoxy; oder

(k) —$OR^{12}$, worin $R^{12}$ ist $C_{3-6}$-Alkenyl, $C_{5-7}$-Cycloalkyl (gegebenenfalls substituiert durch eine oder mehrere $C_{1-4}$-Alkylgruppen), —$CH_2CCl_3$ oder Furanylmethyl;

n ist 1 oder 2;

W ist geradkettiges oder verzweigtes $C_{1-7}$-Alkylen;

X ist cis- oder trans-CH=CH— oder —$CH_2CH_2$—;

Y ist eine gesättigte heterocyclische Aminogruppe (verknüpft mit dem Cyclopentanring über das Stickstoffatom), welche 5 bis 8 Ringglieder hat und (a) gegebenenfalls im Ring —O—, —S—, $—SO_2—$ oder $—NR^{13}—$ enthält (worin $R^{13}$ ein Wasserstoffatom, $C_{1-7}$-Alkyl oder Aralkyl mit einem $C_{1-4}$-Alkylteil ist); und/oder (b) gegebenenfalls substituiert ist durch eine oder mehrere $C_{1-4}$-Alkylgruppen;

$R^2$ ist (i) geradkettiges oder verzweigtes $C_{1-5}$-Alkyl substituiert durch (a) Phenyl [gegebenenfalls substituiert durch $C_{1-6}$-Alkyl, $C_{5-7}$-Cycloalkyl, Phenylalkyl mit einem $C_{1-3}$-Alkylteil, Thienyl, Phenyl (gegebenenfalls substituiert durch $C_{1-4}$-Alkyl, $C_{1-4}$-Alkoxy oder Phenyl), Benzoyl (gegebenenfalls substituiert durch $C_{1-4}$-Alkyl, $C_{1-4}$-Alkoxy, Halogen, Trifluormethyl oder Phenyl) oder $C_{5-7}$-Cycloalkanoyl], (b) Thienyl [gegebenenfalls substituiert durch $C_{5-7}$-Cycloalkyl oder Phenyl (gegebenenfalls substituiert durch $C_{1-3}$-Alkyl, $C_{1-3}$-Alkoxy oder Halogen)], oder (c) Naphthyl (gegebenenfalls substituiert durch $C_{1-4}$-Alkyl oder $C_{1-4}$-Alkoxy), oder (ii) Cinnamyl (gegebenenfalls substituiert durch Benzoyl); und der physiologisch annehmbaren Salze und Solvate davon, dadurch gekennzeichnet:

(a) daß man die entsprechende Verbindung, worin $R^1$ —OH ist, verestert, oder zur Herstellung einer Verbindung, worin $R^1$ eine Gruppe vom Typ (c) und A —S— ist, die entsprechende Verbindung, worin $R^1$ —SH ist, mit dem Halogenid $R^5BCH_2Hal$ umsetzt;

(b) daß man die entsprechende Verbindung, worin X eine Acetylengruppe ist, reduziert;

(c) daß man die entsprechende Verbindung, worin $R^3$ Phenyl, substituiert durch Amino ist, reduziert;

(d) daß man zur Herstellung einer Verbindung, worin X $—CH_2CH_2—$ ist, eine entsprechende Verbindung, worin X —CH=CH— ist, katalytisch hydriert;

(e) daß man zur Herstellung einer Verbindung der Formel (1b) eine entsprechende Hydroxyverbindung der Formel (4)

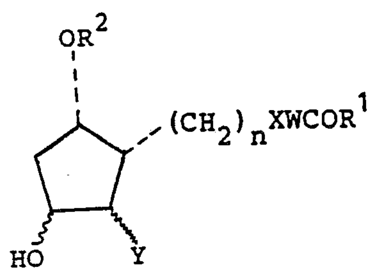

(4)

oxidiert;

(f) daß man zur Herstellung einer Verbindung der Formel (1b), worin $R^1$ $—OCH_2SCH_3$ ist, die entsprechende Verbindung der Formel (4), worin $R^1$ —OH ist, gleichzeitig oxidiert und verestert; oder

(g) daß man zur Herstellung eines Salzes eine Verbindung der Formel (1a) oder (1b) mit einer Säure, oder, wenn $R^1$ eine —COOH-Gruppe enthält, mit einer Base behandelt.

2. Verfahren gemäß Anspruch 1, worin Y in dem Produkt Morpholino oder Piperidino ist.

3. Verfahren gemäß einem der vorhergehenden Ansprüche, worin X in dem Produkt cis-CH=CH— ist.

4. Verfahren gemäß einem der vorhergehenden Ansprüche, dadurch gekennzeichnet, daß in dem Produkt n = 2 und W ist $—CH_2CH_2—$ oder $—CH_2CH_2CH_2CH_2—$.

5. Verfahren gemäß einem der vorhergehenden Ansprüche, dadurch gekennzeichnet, daß $R^2$ in dem Produkt eine Benzylgruppe ist, worin die Phenylgruppe durch Thienyl oder Phenyl substituiert ist, welch letztere Phenylgruppe gegebenenfalls durch $C_{1-4}$-Alkyl oder $C_{1-4}$-Alkoxy substituiert ist.

6. Verfahren gemäß einem der vorhergehenden Ansprüche, dadurch gekennzeichnet, daß $R^1$ in dem Produkt $—OCH_2OCOCH_3$, $—OCH_2SCH_3$, $—O(CH_2)_3NHCOCH_3$, $—OCH_2CONH_2$, 4-Acetamidophenoxy oder Allyloxy ist.

7. Verfahren gemäß Anspruch 1, dadurch gekennzeichnet, daß das Produkt eine Verbindung der Formel (1a) oder (1b) ist, worin:

$R^1$ ist $—OCH_2OCOCH_3$, $—OCH_2SCH_3$, $—O(CH_2)_3NHCOCH_3$, $—OCH_2CONH_2$, 4-Acetamidophenoxy oder Allyloxy,

W ist $—CH_2CH_2$,

n = 2,

X = cis-CH=CH—

Y ist Morpholino oder Piperidino in Verbindungen der Formel (1b) oder ist Piperidino in Verbindungen der Formel (1a), und

$R^2$ ist Benzyl, worin die Phenylgruppe durch Phenyl, Tolyl oder Methoxyphenyl substituiert ist, oder ein physiologisch annehmbares Salz oder Solvat davon.

8. Verfahren gemäß einem der vorhergehenden Ansprüche, dadurch gekennzeichnet, daß das die $—(CH_2)_nXWCOR^1$-Gruppe tragende Kohlenstoffatom in dem Produkt in der R-Konfiguration vorliegt.

9. Verfahren gemäß einem der vorhergehenden Ansprüche, dadurch gekennzeichnet, daß das Produkt eine Verbindung der Formel (1a) ist.

10. Verfahren zur Herstellung einer pharmazeutischen Zusammensetzung, umfassend eine Verbindung der Formel (1), wie in einem der vorhergehenden Ansprüche definiert, dadurch gekennzeichnet, daß diese Verbindung mit einem oder mehreren pharmazeutischen Trägern formuliert wird.

11. Verfahren gemäß Anspruch 10, dadurch gekennzeichnet, daß eine Einheitsdosis der Verbindung der Formel (1) als Tablette oder Flüssigkeit zur Injektion formuliert wird oder die Verbindung der Formel (1) als Aerosol, Lösung oder Inhalationspatrone zum Inhalieren einer abgemessenen Menge der Verbindung formuliert ist.

**Revendications pour les Etats contractants: BE CH DE FR GB IT LI LU NL SE**

1. Composés de formules développées (1a) et (1b)

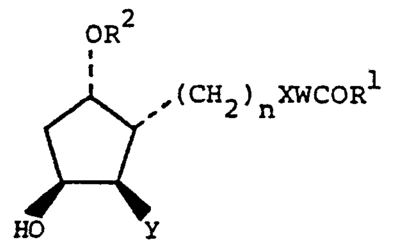

(1a)

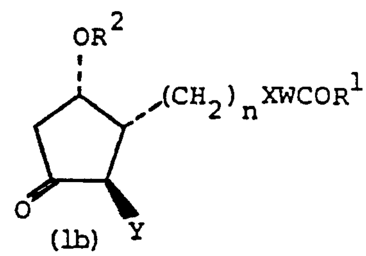

(1b)

dans lesquelles $R^1$ est

(a) $—AR^3$ dans laquelle A est —O— ou —S— et $R^3$ est phényle [éventuellement substitué par un alcoyle en $C_1$ à $C_4$, un alcoxy en $C_1$ à $C_4$, un alcanoyle en $C_1$ à $C_4$, un méthylthio, un méthylsulfinyle, un méthylsulfonyle, un halogène, $—CO_2R^4$ (dans laquelle $R^4$ est un atome d'hydrogène, un alcoyle en $C_1$ à $C_4$ ou phényle), $—NHCOR^4$, $—CONR^5R^6$ (dans laquelle $R^5$ et $R^6$ peuvent être identiques ou différents et sont chacun un atome d'hydrogène ou un alcoyle en $C_1$ à $C_4$), alcoylsulfonylamino en $C_1$ à $C_4$, formyle, nitro, cyano, phényle ou $NR^5R^6$];

(b) $—OCH_2COR^7$ dans laquelle $R^7$ est phényle (éventuellement substitué par un atome d'halogène, un alcoyle en $C_1$ à $C_4$ ou un alcoxy en $C_1$ à $C_4$) ou $—NH_2$;

(c) $—A(CH_2)_mBR^5$ dans laquelle A est —O— ou —S—, m est égal à 1 à 3 et B est —O— ou —S—, pourvu que, quand m est égal à 1, $R^5$ ne soit pas un atome d'hydrogène;

(d) $—A(CH_2)_pR^8$ dans laquelle A est —O— ou —S—, p est 2 ou 3 et $R^8$ est un groupe dialcoylamino en $C_1$ à $C_4$, morpholino, pipéridino, pyrrolidino, acétylamino ou benzoylamino fixé par l'azote;

(e) $—OCH(CH_2N(CH_3)_2)_2$;

(f)

$R^9$
|
$—ACHCO_2R^5$

dans laquelle A est —O— ou —S— et $R^9$ est un atome d'hydrogène, méthyle ou phényle;

(g) $—OCH_2OCOR^{10}$ dans laquelle $R^{10}$ est alcoyle en $C_1$ à $C_4$, méthoxy ou phényle;

(h) $—OCH_2SCOR^{11}$ dans laquelle $R^{11}$ est alcoyle en $C_1$ à $C_4$;

(i) pyridinyloxy ou pyridinylthio;

(j) 1-(acétyloxy)éthoxy, (acétyloxy)phénylméthoxy, tétrahydro-5-oxo-2-furanoyloxy, tétrahydro-2-oxo-3-furanyloxy, triphénylméthoxy ou diphénylméthoxy; ou

(k) $—OR^{12}$ dans laquelle $R^{12}$ est alcényle en $C_3$ à $C_6$, cycloalcoyle en $C_5$ à $C_7$ (éventuellement substitué par un ou par plusieurs groupes alcoyle en $C_1$ à $C_4$), $—CH_2CCl_3$ ou furanylméthyle;

n est égal à 1 ou à 2;

W est un alcoylène linéaire ou ramifié en $C_1$ à $C_7$;

X est —CH=CH— cis ou trans ou $—CH_2CH_2—$;

Y est un groupe amino hétérocyclique saturé (fixé au cycle cyclopentanique par l'atome d'azote) qui a de 5 à 8 chaînons et (a) qui contient éventuellement dans le cycle —O—, —S—, $—SO_2—$, ou $—NR^{13}—$ (dans laquelle $R^{13}$ est un atome d'hydrogène, un alcoyle en $C_1$ à $C_7$, ou un aralcoyle ayant une portion alcoylique en $C_1$ à $C_4$); et/ou (b) est éventuellement substitué par un ou plusieurs groupes alcoyle en $C_1$ à $C_4$;

$R^2$ est (i) un alcoyle linéaire ou ramifié en $C_1$ à $C_5$, substitué par (a) un phényle [éventuellement substitué par un alcoyle en $C_1$ à $C_6$, un cycloalcoyle en $C_5$ à $C_7$, un phénylalcoyle ayant une portion alcoylique en $C_1$ à $C_3$, un thiényle, un phényle (éventuellement substitué par un alcoyle en $C_1$ à $C_4$, par un alcoxy en $C_1$ à $C_4$ ou par un phényle), un benzoyle (éventuellement substitué par un alcoyle en $C_1$ à $C_4$, par

un alcoxy en $C_1$ à $C_4$, par un halogène, par un trifluorométhyle ou par un phényle) ou cycloalcanoyle en $C_5$ à $C_7$], (b) un thiényle [éventuellement substitué par un cycloalcoyle en $C_5$ à $C_7$ ou par un phényle (éventuellement substitué par un alcoyle en $C_1$ à $C_3$, par un alcoxy en $C_1$ à $C_3$ ou par un halogène], ou (c) un naphtyle (éventuellement substitué par un alcoyle en $C_1$ à $C_4$ ou par un alcoxy en $C_1$ à $C_4$), ou (ii) un cinnamyle (éventuellement substitué par un benzoyle); et les sels physiologiquement acceptables et produits solvatés de ceux-ci.

2. Composés tels que revendiqués à la revendication 1, dans lesquels Y est morpholino ou pipéridino.

3. Composés tels que revendiqués suivant l'une quelconque des revendications précédentes, dans lesquels X est —CH=CH— cis.

4. Composés tels que revendiqués suivant l'une quelconque des revendications précédentes, dans lesquelles n est 2 et W est $—CH_2CH_2$ ou $—CH_2CH_2CH_2CH_2—$.

5. Composés tels que revendiqués suivant l'une quelconque des revendications précédentes, dans lesquels $R^2$ est un groupe benzyle dans lequel le groupe phényle est substitué par un thiényle ou par un phényle, ce dernier phényle étant éventuellement substitué par un alcoyle en $C_1$ à $C_4$ ou par un alcoxy en $C_1$ à $C_4$.

6. Composés tels que revendiqués suivant l'une quelconque des revendications précédentes, dans lesquels $R^1$ est $—OCH_2OCOCH_3$, $—OCH_2SCH_3$, $—O(CH_2)_3NHCOCH_3$, $—OCH_2CONH_2$, 4-acétamidophénoxy ou allyloxy.

7. Composés tels que revendiqués à la revendication 1, dans lesquels:

$R^1$ est $—OCH_2OCOCH_3$, $—OCH_2SCH_3$, $—O(CH_2)_3NHCOCH_3$, $—OCH_2CONH_2$, 4-acétamidophénoxy ou allyloxy,

W est $—CH_2CH_2—$,

n est égal à 2,

X est —CH=CH— cis,

Y est morpholino ou pipéridino dans les composés de formule (1b) ou est pipéridino dans les composés de formule (1a), et

$R^2$ est benzyle dans lequel le groupe phényle est substitué par un phényle, un tolyle ou un méthoxyphényle, et leurs sels physiologiquement acceptables et leurs produits solvatés.

8. Composés tels que revendiqués à l'une quelconque des revendications précédentes dans lesquels l'atome de carbone portant le groupe $—(CH_2)_nXWCOR^1$ est en la configuration R—.

9. Composés tels que revendiqués suivant l'une quelconque des revendications précédentes, ces composés ayant la formule (1a).

10. Composition pharmaceutique comprenant un composé tel que revendiqué à l'une quelconque des revendications précédentes et un ou plusieurs véhicules pharmaceutiques.

11. Procédé de préparation d'un composé tel que revendiqué à la revendication 1, qui consiste:

(a) à estérifier le composé correspondant dans lequel $R^1$ est —OH ou, pour la préparation d'un composé dans lequel $R^1$ est un groupe de type (c) et A est —S—, à faire réagir le composé correspondant dans lequel $R^1$ est —SH sur un halogénure, $R^5BCH_2Hal$;

(b) à réduire le composé correspondant dans lequel X est un groupe acétylène;

(c) à réduire le composé correspondant dans lequel $R^3$ est un phényle substitué par un azido pour préparer un composé dans lequel $R^3$ est phényle substitué par un amino;

(d) pour la préparation d'un composé dans lequel X est $—CH_2CH_2—$, à hydrogéner catalytiquement un composé correspondant dans lequel X est —CH=CH—;

(e) pour la préparation d'un composé de formule (1b), à oxyder un composé hydroxylé correspondant de formule (4)

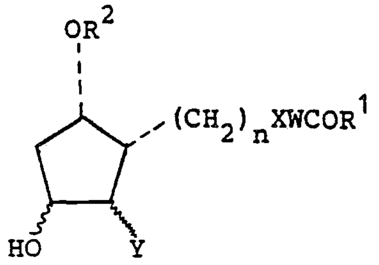

(4)

(f) pour la préparation d'un composé de formule (1b), dans laquelle $R^1$ est $—OCH_2SCH_3$, à oxyder et à estérifier simultanément le composé correspondant de formule (4) dans laquelle $R^1$ est —OH; ou

(g) pour la préparation d'un sel, à traiter un composé de formule (1a) ou (1b) par un acide, ou, quand $R^1$ contient un groupe —COOH, par une base.

**Revendications pour l'Etat contractant: AT**

1. Procédé de préparation d'un composé de formules développées (1a) ou (1b)

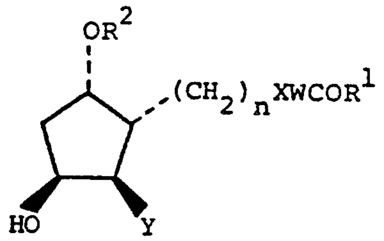

(1a)

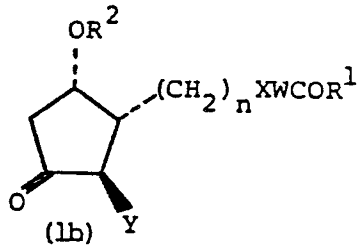

(1b)

dans lesquelles $R^1$ est

(a) $—AR^3$ dans laquelle A est —O— ou —S— et $R^3$ est phényle [éventuellement substitué par un alcoyle en $C_1$ à $C_4$, un alcoxy en $C_1$ à $C_4$, un alcanoyle en $C_1$ à $C_4$, un méthylthio, un méthylsulfinyle, un méthylsulfonyle, un halogène, $—CO_2R^4$ (dans laquelle $R^4$ est un atome d'hydrogène, un alcoyle en $C_1$ à $C_4$ ou phényle), $—NHCOR^4$, $—CONR^5R^6$ (dans laquelle $R^5$ et $R^6$ peuvent être identiques ou différents et sont chacun un atome d'hydrogène ou un alcoyle en $C_1$ à $C_4$), alcoylsulfonylamino en $C_1$ à $C_4$, formyle, nitro, cyano, phényle ou $NR^5R^6$];

(b) $—OCH_2COR^7$ dans laquelle $R^7$ est phényle (éventuellement substitué par un atome d'halogène, un alcoyle en $C_1$ à $C_4$ ou un alcoxy en $C_1$ à $C_4$) ou $—NH_2$;

(c) $—A(CH_2)_mBR^5$ dans laquelle A est —O— ou —S—, m est égal à 1 à 3 et B est —O— ou —S—, pourvu que, quand m est égal à 1, $R^5$ ne soit pas un atome d'hydrogène;

(d) $—A(CH_2)_pR^8$ dans laquelle A est —O— ou —S—, p est 2 ou 3 et $R^8$ est un groupe dialcoylamino en $C_1$ à $C_4$, morpholino, pipéridino, pyrrolidino, acétylamino ou benzoylamino fixé par l'azote;

(e) $—OCH(CH_2N(CH_3)_2)_2$;

(f)

$$—A\overset{R^9}{\overset{|}{C}}HCO_2R^5$$

dans laquelle A est —O— ou —S— et $R^9$ est un atome d'hydrogène, méthyle ou phényle;

(g) $—OCH_2OCOR^{10}$ dans laquelle $R^{10}$ est alcoyle en $C_1$ à $C_4$, méthoxy ou phényle;

(h) $—OCH_2SCOR^{11}$ dans laquelle $R^{11}$ est alcoyle en $C_1$ à $C_4$;

(i) pyridinyloxy ou pyridinylthio;

(j) 1-(acétyloxy)éthoxy, (acétyloxy)phénylméthoxy, tétrahydro-5-oxo-2-furanoyloxy, tétrahydro-2-oxo-3-furanyloxy, triphénylméthoxy ou diphénylméthoxy; ou

(k) $—OR^{12}$ dans laquelle $R^{12}$ est alcényle en $C_3$ à $C_6$, cycloalcoyle en $C_5$ à $C_7$ (éventuellement substitué par un ou par plusieurs groupes alcoyle en $C_1$ à $C_4$), $—CH_2CCl_3$ ou furanylméthyle;

n est égal à 1 ou à 2;

W est un alcoylène linéaire ou ramifié en $C_1$ à $C_7$;

X est —CH=CH— cis ou trans ou $—CH_2CH_2—$;

Y est un groupe amino hétérocyclique saturé (fixé au cycle cyclopentanique par l'atome d'azote) qui a de 5 à 8 chaînons et (a) qui contient éventuellement dans le cycle —O—, —S—, $—SO_2—$, ou $—NR^{13}—$ (dans laquelle $R^{13}$ est un atome d'hydrogène, un alcoyle en $C_1$ à $C_7$, ou un aralcoyle ayant une portion alcoylique en $C_1$ à $C_4$); et/ou (b) est éventuellement substitué par un ou plusieurs groupes alcoyle en $C_1$ à $C_4$;

$R^2$ est (i) un alcoyle linéaire ou ramifié en $C_1$ à $C_5$, substitué par (a) un phényle [éventuellement. substitué par un alcoyle en $C_1$ à $C_6$, un cycloalcoyle en $C_5$ à $C_7$, un phénylalcoyle ayant une portion alcoylique en $C_1$ à $C_3$, un thiényle, un phényle (éventuellement substitué par un alcoyle en $C_1$ à $C_4$, par un alcoxy en $C_1$ à $C_4$ ou par un phényle), un benzoyle (éventuellement substitué par un alcoyle en $C_1$ à $C_4$, par un alcoxy en $C_1$ à $C_4$, par un halogène, par un trifluorométhyle ou par un phényle) ou cycloalcanoyle en $C_5$ à $C_7$], (b) un thiényle [éventuellement substitué par un cycloalcoyle en $C_5$ à $C_7$ ou par un phényle (éventuellement substitué par un alcoyle en $C_1$ à $C_3$, par un alcoxy en $C_1$ à $C_3$ ou par un halogène], ou (c) un naphtyle (éventuellement substitué par un alcoyle en $C_1$ à $C_4$ ou par un alcoxy en $C_1$ à $C_4$), ou (ii) un cinnamyle (éventuellement substitué par un benzoyle); et de leurs sels physiologiquement acceptables et produits solvatés de ceux-ci, ce procédé consistant:

(a) à estérifier le composé correspondant dans lequel $R^1$ est —OH ou, pour la préparation d'un composé dans lequel $R^1$ est un groupe de type (c), et A est —S—, à faire réagir le composé correspondant dans lequel $R^1$ est —SH sur un halogénure, $R^5BCH_2Hal$;

(b) à réduire le composé correspondant dans lequel X est un groupe acétylène;

(c) à réduire le composé correspondant dans lequel $R^3$ est un phényle substitué par un azido pour préparer un composé dans lequel $R^3$ est phényle substitué par un amino;

(d) pour la préparation d'un composé dans lequel X est $—CH_2CH_2—$, à hydrogéner catalytiquement un composé correspondant dans lequel X est —CH=CH—;

(e) pour la préparation d'un composé de formule (1b), à oxyder un composé correspondant de formule (4)

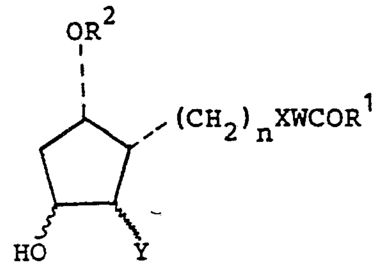

(4)

(f) pour la préparation d'un composé de formule (1b), dans laquelle $R^1$ est $—OCH_2SCH_3$, à oxyder et à estérifier simultanément le composé correspondant de formule (4) dans laquelle $R^1$ est —OH; ou

(g) pour la préparation d'un sel, à traiter un composé de formule (1a) ou (1b) par un acide, ou quand $R^1$ contient un groupe —COOH, par une base.

2. Procédé tel que revendiqué à la revendication 1, dans lequel Y dans le produit est morpholino ou pipéridino.

3. Procédé tel que revendiqué suivant l'une quelconque des revendications précédentes, dans lequel X dans le produit est —CH=CH— cis.

4. Procédé tel que revendiqué suivant l'une quelconque des revendications précédentes, dans lequel dans le produit n est 2 et W est $—CH_2CH_2—$ ou $—CH_2CH_2CH_2CH_2—$.

5. Procédé tel que revendiqué suivant l'une quelconque des revendications précédentes, dans lequel $R^2$ dans le produit est un groupe benzyle dont le groupe phényle est substitué par un thiényle ou par un phényle, ce dernier phényle étant éventuellement substitué par un alcoyle en $C_1$ à $C_4$ ou par un alcoxy en $C_1$ à $C_4$.

6. Procédé suivant l'une quelconque des revendications précédentes, dans lequel $R^1$ dans le produit est $—OCH_2OCOCH_3$, $—OCH_2SCH_3$, $—O(CH_2)_3NHCOCH_3$, $—OCH_2CONH_2$, 4-acétamidophénoxy ou allyloxy.

7. Procédé tel que revendiqué suivant la revendication 1, dans lequel le produit est un composé de formule (1a) ou (1b) dans lesquelles:

$R^1$ est $—OCH_2OCOCH_3$, $—OCH_2SCH_3$, $—O(CH_2)_3NHCOCH_3$, $—OCH_2CONH_2$, 4-acétamidophénoxy ou allyloxy,

W est $—CH_2CH_2—$,

n est égal à 2,

X est —CH=CH— cis,

Y est morpholino ou pipéridino dans les composés de formule (1b) ou est pipéridino dans les composés de formule (1a), et

$R^2$ est benzyle dans lequel le groupe phényle est substitué par un phényle, un tolyle ou un méthoxyphényle, ou un sel physiologiquement acceptable et son produit solvaté.

8. Procédé tel que revendiqué suivant l'une quelconque des revendications précédentes, dans lequel l'atome de carbone portant le groupe $—(CH_2)_nXWCOR^1$ est en la configuration R—.

9. Procédé tel que revendiqué suivant l'une quelconque des revendications précédentes, dans lequel le produit est un composé de formule (1a).

10. Procédé de fabrication d'une composition pharmaceutique comprenant un composé de formule (1) tel que défini dans l'une quelconque des revendications précédentes, qui consiste à formuler ce composé avec un ou plusieurs véhicules pharmaceutiques.

11. Procédé tel que revendiqué à la revendication 10, qui consiste à formuler une dose unitaire du composé de formule (1) sous la forme d'un comprimé ou d'un liquide d'injection, ou qui consiste à formuler le composé de formule (1) en aérosol, en solution ou en cartouche d'inhalation pour l'inhalation d'une quantité déterminée du composé.